# FASCICULE DE BREVET EUROPEEN

(11) **EP 2 257 639 B1**
(45) Date de publication et mention de la délivrance du brevet: **08.05.2013**
(21) Numéro de dépôt: 09726525.0
(22) Date de dépôt: 10.03.2009
(51) Int. Cl.: C12Q 1/68, C12Q 1/70

(54) **PROCEDE POUR LE DIAGNOSTIC OU PRONOSTIC IN VITRO DU CANCER DU TESTICULE**
VERFAHREN ZUR IN-VITRO-DIAGNOSE ODER PROGNOSE VON HODENKREBS
METHOD FOR THE IN VITRO DIAGNOSIS OR PROGNOSIS OF TESTICULAR CANCER

(30) Priorité: 12.03.2008 FR 0851621
(43) Date de publication de la demande: 08.12.2010
(73) Titulaire: Biomérieux, 69280 Marcy L'etoile (FR)
(72) Inventeur: GIMENEZ, Juliette, F-69300 Caluire et Cuire (FR); MONTGIRAUD, Cécile, F-69007 Lyon (FR); MALLET, François, F-69100 Villeurbanne (FR)
(86) Numéro de dépôt international: PCT/FR2009/050388
(87) Numéro de publication internationale: WO 2009/122052

(56) Documents cités:
- US-A- 5 858 723
- YI J-M ET AL: "Expression of the human endogenous retrovirus HERV-W family in various human tissues and cancer cells" JOURNAL OF GENERAL VIROLOGY, SOCIETY FOR GENERAL MICROBIOLOGY, SPENCERS WOOD, GB, vol. 85, no. Pt 5, 1 mai 2004 (2004-05-01), pages 1203-1210, XP002417878 ISSN: 0022-1317
- MATOUSKOVA ET AL: "CpG methylation suppresses transcriptional activity of human syncytin-1 in non-placental tissues" EXPERIMENTAL CELL RESEARCH, ACADEMIC PRESS, US, vol. 312, no. 7, 15 avril 2006 (2006-04-15), pages 1011-1020, XP005346075 ISSN: 0014-4827
- LAVIE LAURENCE ET AL: "CpG methylation directly regulates transcriptional activity of the human endogenous retrovirus family HERV-K(HML-2)." JOURNAL OF VIROLOGY JAN 2005, vol. 79, no. 2, janvier 2005 (2005-01), pages 876-883, XP002500945 ISSN: 0022-538X

## Description

Le cancer du testicule représente 1 à 2% des cancers chez l'homme, et 3,5% des tumeurs urologiques. C'est la tumeur la plus fréquente chez l'homme jeune, rare avant 15 ans et après 50 ans. Le risque est plus élevé chez les patients séropositifs pour le virus HIV. Le séminome est la forme la plus fréquente du cancer du testicule (40%), mais de nombreux autres types de cancer existent parmi lesquels le carcinome embryonnaire (20%), le tératocarcinome (30%) et le choriocarcinome (1%).

Le diagnostic du cancer du testicule est d'abord clinique : il se présente souvent sous la forme d'une tuméfaction dure et irrégulière du testicule. L'échographie confirme la tumeur intra-testiculaire et l'échographie Doppler met en évidence l'augmentation de la vascularisation dans la tumeur. Dans certains cas, un examen par résonance magnétique (IRM testiculaire) peut être utile. Un scanner thoracique, abdominal et pelvien permet de rechercher une extension ganglionnaire du cancer. Un prélèvement sanguin pour le dosage des marqueurs tumoraux est quasi-systématique. Il permet d'orienter le diagnostic du type de tumeur. Deux marqueurs tumoraux principaux sont utilisés et dosés dans le sang : la β-HCG et l'α-foetoprotéine. Mais ces marqueurs ne sont pas très spécifiques et de plus leur concentration à des niveaux physiologiques ne signifie pas une absence de tumeur. A ce jour, le diagnostic et le pronostic finaux sont posés après l'ablation du testicule atteint (orchidectomie) qui constitue le premier stade du traitement. Ensuite, en fonction du type de cancer et de son stade, un traitement complémentaire par radiothérapie ou chimiothérapie est appliqué. Il existe donc un réel besoin de pouvoir disposer de marqueurs spécifiques du cancer du testicule qui, de plus, permettent d'établir un diagnostic et un pronostic les plus précoces possible.

L'événement rare que représente l'infection d'une cellule de la lignée germinale par un provirus exogène conduit à l'intégration dans le génome de l'hôte d'un ADN proviral ou provirus, celui-ci devenant partie intégrante du patrimoine génétique de l'hôte. Ce provirus endogène (HERV) est donc transmissible à la génération suivante de façon mendélienne. On estime qu'il existe environ une centaine de familles HERV représentant environ 8% du génome humain. Chacune des familles compte de quelques dizaines à des milliers de loci, qui sont le résultat de rétrotranspositions intracellulaires de copies transcriptionnellement actives. Les loci des familles HERV contemporaines sont tous sont défectifs pour la réplication, ce qui signifie la perte des propriétés infectieuses et implique donc un mode de transmission exclusivement vertical (mendélien).

L'expression des HERV a été particulièrement étudiée dans trois contextes spécifiques, la placentation, l'auto-immunité et le cancer, qui sont associés à la différentiation cellulaire ou à la modulation de l'immunité. Il a ainsi été montré que la glycoprotéine d'enveloppe du locus ERVWE1 de la famille HERV-W est impliquée dans le processus de fusion aboutissant à la formation du syncytiotrophoblaste. Il a par ailleurs été suggéré que la protéine Rec, variant d'épissage du gène *env* de HERV-K, pourrait avoir une implication dans le processus de tumorigenèse testiculaire. Cependant, il n'a pas encore été répondu à la question suivante : les HERV sont-ils des acteurs ou des marqueurs dans des contextes pathologiques ?

Les présents inventeurs ont maintenant découvert et démontré que des séquences d'acides nucléiques, appartenant à des loci de la famille HERV-W, sont associées au cancer du testicule et que ces séquences sont des marqueurs moléculaires de la pathologie. Les séquences identifiées sont des séquences de promoteurs rétroviraux U3 de LTRs 5' (LTR ou « Long Terminal Repeat » selon la terminologie anglo-saxonne) qui sont hypométhylées dans un échantillon biologique cancéreux.

Chez les mammifères l'ADN peut être méthylé au niveau des cytosines précédant une guanine (doublet CpG). Il s'agit du transfert d'un groupement méthyle du S-adénosyl méthionine à un résidu cytosine pour former du 5-méthylcytosine. La méthylation des doublets CpG situés dans une séquence promotrice, conduit généralement à une sous expression, voire à une absence d'expression, du gène *associé. A contrario,* si les doublets CpG contenus dans une séquence promotrice sont hypométhylés, l'expression du gène associé est favorisée. Le rôle de la méthylation dans la carcinogenèse a été récemment étudié. Ainsi, une hyperméthylation, au niveau de doublets CpG peut conduire à la sous expression de gène suppresseur de tumeur, alors qu'au contraire une hypométhylation de doublets CpG peut provoquer l'activation de proto-oncogènes.

La présente invention a donc pour objet un procédé pour le diagnostic ou le pronostic, *in vitro,* du cancer du testicule dans un échantillon biologique issu d'un patient suspecté d'être atteint d'un cancer du testicule, caractérisé en ce qu'il comprend une étape de détection du niveau de méthylation desdinucléotides CpG dans au moins une séquence cible d'ADN génomique de l'échantillon, la séquence cible étant choisie parmi au moins une des séquences identifiées en SEQ ID NOs : 1 à 7 ou parmi au moins une séquence qui présente au moins 99% d'identité, de préférence au moins 99,5% d'identité et avantageusement au moins 99,6% d'identité avec une des séquences identifiées en SEQ ID NOs : 1 à 7 et leurs séquences complémentaires.

Le pourcentage d'identité décrit ci-dessus a été déterminé en prenant en considération la diversité nucléotidique dans le génome. Il est connu que la variabilité nucléotidique est plus élevée dans les régions du génome riches en séquences répétées que dans les régions ne contenant pas de séquences répétées. A titre d'exemple, Nickerson D. *A. et al.,*^{[1]} ont montré une diversité d'environ 0,3% (0,32%) dans des régions contenant des séquences répétées.

Les séquences SEQ ID NOs : 1 à 6 correspondent respectivement aux séquences des promoteurs rétroviraux U3 des loci HW4TT, HW2TT, HW13TT, HWXTT, HW21TT, ERVWE1 et la SEQ ID NO : 7 correspond à la séquence de l'activateur plus la séquence de la région U3 de ERVWE1.

L'échantillon du patient comprendra généralement des cellules (telles que les cellules testiculaires). Elles peuvent être présentes dans un échantillon de tissu (tel que le tissu testiculaire) ou être trouvées dans la circulation. En général, l'échantillon est un extrait tissulaire de testicule ou un fluide biologique, tel que le sang, le sérum, le plasma, l'urine, ou encore le liquide séminal.

Plus particulièrement le procédé comprend :
(i) une extraction de l'ADN génomique à analyser dans l'échantillon,
(ii) un traitement de l'ADN génomique extrait avec un ou plusieurs réactifs pour convertir les bases cytosine, des dinucléotides CpG, non méthylées en position 5 en uracile,
(iii) au moins une amplification de l'ADN traité par mise en contact avec au moins deux amorces,
(iv) la détermination, sur la base de la présence ou de l'absence de méthylation des cytosines des dinucléotides CpG, d'un état de méthylation de ladite séquence cible ou d'une valeur qui reflète l'état de méthylation de la séquence cible, par exemple le rapport nombre de cytosines méthylées des dinucléotides CpG / nombre total de cytosines des dinucléotides CpG. En particulier si le rapport, correspondant à un pourcentage de méthylation, est inférieur ou égal à 80%, de préférence inférieur ou égal à 60% et avantageusement inférieur ou égal à 30%, ceci peut être corrélé avec une présomption de cancer du testicule.

Si nécessaire le procédé comprend une deuxième étape d'amplification après l'étape d'amplification décrite en (iii) qui consiste à mettre en contact les amplicons obtenus en (iii) avec au moins deux amorces pour amplifier la séquence cible.

Par séquence cible on entend une séquence ou les séquences d'un ensemble de clones.

La détermination, dans l'ADN, du taux de méthylation est effectuée par toute technique appropriée. L'état ou statut de méthylation d'une séquence ADN peut être établi par des méthodes utilisant des enzymes de restriction sensibles à la méthylation ou par des méthodes impliquant une modification chimique de l'ADN par le bisulfite de sodium, le sulfite d'hydrogène ou le disulfite, de préférence par une solution de bisulfite de sodium qui convertit les cytosines non méthylées en uraciles tout en ne modifiant pas les 5-méthylcytosines. L'analyse de la méthylation peut être effectuée par des méthodes classiques, telles que le séquençage, l'hybridation ou la PCR. Plusieurs méthodes d'analyse utilisent la technique de conversion au bisulfite d'ammonium, telles que la « Bisulfite sequencing PCR » (conversion au bisulfite d'ammonium, amplification de la séquence d'intérêt et séquençage), la « MSP » (« Methylation Specific PCR », PCR spécifique de la méthylation) et la « MSO » (« Methylation Specific Oligonucleotide Microarray ») utilisant des puces ADN spécifiques pour l'ADN modifié. Toutes ces méthodes sont bien connues de l'homme du métier et on peut citer pour illustration Cottrell S. E. ^{[2]}.

Ainsi, dans l'étape (ii) du procédé précité le traitement de l'ADN génomique comprend l'utilisation d'une solution choisie dans le groupe consistant en bisulfite, disulfite, sulfite d'hydrogène et leurs combinaisons ; de préférence une solution de bisulfite de sodium.

Dans un mode de réalisation de l'invention, le procédé de diagnostic et/ou de pronostic, *in vitro,* du cancer du testicule comprend :
(i) une extraction de l'ADN à analyser dans l'échantillon du patient,
(ii) une détermination, dans l'ADN à analyser, du taux (pourcentage) de méthylation des cytosines des dinucléotides CpG compris dans au moins une des séquences ADN identifiées en SEQ ID NOs 1 à 7 ou dans au moins une séquence qui présente au moins 99% d'identité, de préférence au moins 99,5% , avantageusement au moins 99,6% d'identité, avec une séquence identifiée en SEQ ID NOs : 1 à 7, et
(iii) une comparaison du taux (pourcentage) de méthylation des cytosines dans une ou des séquences ADN telle(s) que définie(s) en (ii) avec le taux (pourcentage) de méthylation desdites cytosines de la ou desdites séquence(s) présentes dans l'ADN extrait d'un échantillon biologique non cancéreux ; la détermination d'un taux de méthylation dans l'ADN à analyser inférieur au taux de méthylation dans l'ADN extrait de l'échantillon biologique non cancéreux pouvant être corrélé avec le diagnostic ou le pronostic d'un cancer du testicule.

Par séquence hypométhylée on entend donc une séquence ADN comprenant un ou plusieurs doublets CpG dans laquelle une cytosine d'au moins un dinucléotide ou doublet CpG n'est pas méthylée en position 5 (c'est à dire qui ne contient pas de radical CH₃ à la cinquième position de la base cytosine) par comparaison avec une même séquence ADN issue du même type d'échantillon non cancéreux. Pour déterminer l'état ou statut de méthylation d'une séquence cible on peut effectuer le rapport suivant :
nombre de cytosines méthylées des dinucléotides CpG / nombre total de cytosines des dinucléotides CpG. Si le rapport, correspondant à un pourcentage de méthylation, est inférieur ou égal à 80%, de préférence inférieur ou égal à 60% et avantageusement inférieur ou égal à 30%, ceci peut être corrélé avec une présomption de cancer du testicule.

L'invention a encore pour objet une séquence d'acide nucléique, isolée, consistant en moins une séquence ADN choisie parmi les séquences identifiées en SEQ ID NOs : 1 à 7 ou parmi au moins une séquence qui présente au moins 99% d'identité (de préférence au moins 95.5% ou 95,6% d'identité) avec une des séquences identifiées en SEQ ID NOs : 1 à 7 et leurs séquences complémentaires. Les séquences précitées qui sont associées au cancer du testicule sont utilisées comme marqueurs moléculaires du cancer du testicule.

### Figures :

La figure 1 représente le principe de la méthode d'amplification WTA des ARNs.
La figure 2 représente un schéma synoptique de la nature et de l'enchaînement des différentes étapes de prétraitement des données de puces à ADN selon la méthode RMA.
La figure 3 illustre la nomenclature, la position et la structure des loci HERV-W surexprimés et présentant une perte de méthylation dans le testicule tumoral.
La figure 4 est un histogramme représentant l'accroissement d'expression de cinq loci (HW4TT, HW2TT, HW13TT, HWXTT et HW21TT) respectivement dans trois paires d'échantillons testiculaires (testicule 1, testicule 2 et testicule 3), basé sur une quantification comparative échantillon tumoral/échantillon sain. En abscisse sont représentés les loci et en ordonné sont représentés les facteurs d'accroissement de l'expression entre tissu tumoral et tissu sain.

Les figures 5 à 10 représentent le statut de méthylation de la région U3 de la LTR unique ou de la LTR 5' des différents loci, respectivement HW4TT, HW2TT, HW13TT, HWXTT, HW21TT et ERVWE1 dans le testicule sain (normal) et dans le testicule tumoral issus d'un même patient, après amplification et analyse des séquences obtenues.

### Exemples

### Exemple 1 : Identification de loci HERV-W exprimés dans des tissus cancéreux.

### Méthode :

L'identification de loci HERV-W exprimés repose sur la conception d'une puce à ADN haute densité au format GeneChip proposé par la société Affymetrix. Il s'agit d'une puce à façon, développée spécifiquement, dont les sondes correspondent à des loci HERV-W. Les séquences de la famille HERV-W ont été identifiées à partir de la banque de données nucléiques GenBank en utilisant l'algorithme Blast (Altschul et al. 1990) avec la séquence du locus ERVWE1, situé sur le chromosome 7 en 7q21.2 et codant pour la protéine dénommée syncytine. Les séquences homologues à HERV-W ont été comparées à une bibliothèque contenant des séquences de référence de la famille HERV-W (ERVWE1) découpées en régions fonctionnelles (LTR, *gag, pol* et *env*) en utilisant le logiciel RepeatMasker (A.F.A. Smit and P. Green). Ces éléments constituent la banque HERVgDB.

Les sondes composant la puce haute densité ont été définies sur un critère d'unicité de leurs séquences dans la banque HERVgDB. Les LTR provirales et solitaires HERV-W contenues dans la banque HERVgDB ont été extraites. Chacune de ces séquences, a été décomposée en un ensemble de séquences de 25 nucléotides (25-mers) la composant, soit autant de sondes potentielles. L'évaluation de l'unicité de chaque sonde a été réalisée par recherche de similarité avec l'ensemble des 25-mers générés pour toutes les LTRs de la famille considérée. Ceci a permis d'identifier l'ensemble des 25-mers d'occurrence unique pour chaque famille d'HERV. Ensuite, certains de ces 25-mers ont été retenus comme sondes. Pour chaque région cible U3 ou U5, un jeux de sondes a été constitué à partir des sondes identifiées comme uniques.

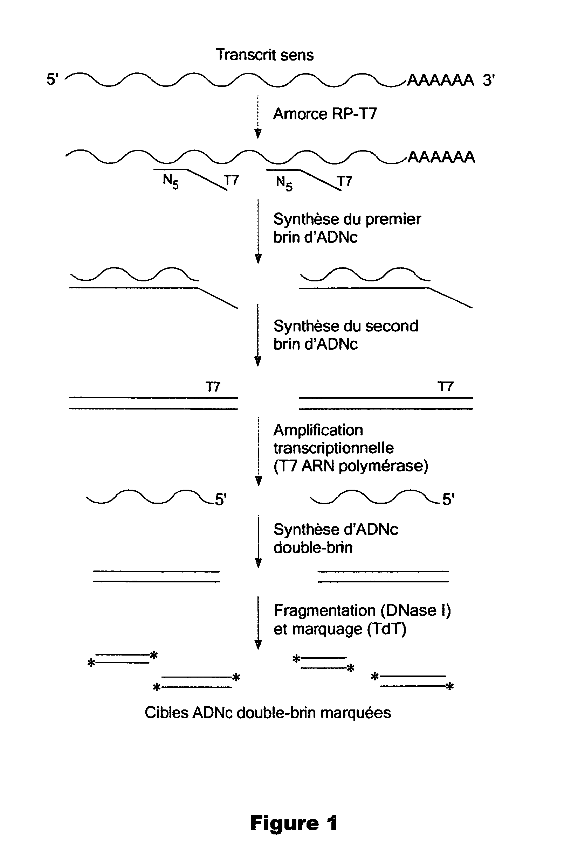

Les échantillons analysés à l'aide de la puce haute-densité HERV correspondent à des ARN extraits de tumeurs et aux ARN extraits des tissus sains adjacents à ces tumeurs. Les tissus analysés sont : l'utérus, le colon, le poumon, le sein, le testicule, la prostate et l'ovaire. Des ARN placentaires (tissu sain uniquement) ont également été analysés. Pour chaque échantillon, 400 ng d'ARN totaux ont été amplifiés à l'aide d'une méthode transcriptionnelle non biaisée connue sous le nom de WTA. Le principe de l'amplification WTA est le suivant: des amorces (RP-T7) comprenant une séquence aléatoire et une séquence promotrice T7 sont hybridées sur les transcrits ; des ADNc double-brin sont synthétisés et servent de matrice à une amplification transcriptionnelle par la T7 ARN polymérase ; les ARN anti-sens générés sont convertis en ADNc double brin qui sont ensuite fragmentés et marqués par introduction d'analogues nucléotidiques biotinylés aux niveaux des extrémités 3'OH par la *terminale transferase* (TdT) (cf. : Figure 1).

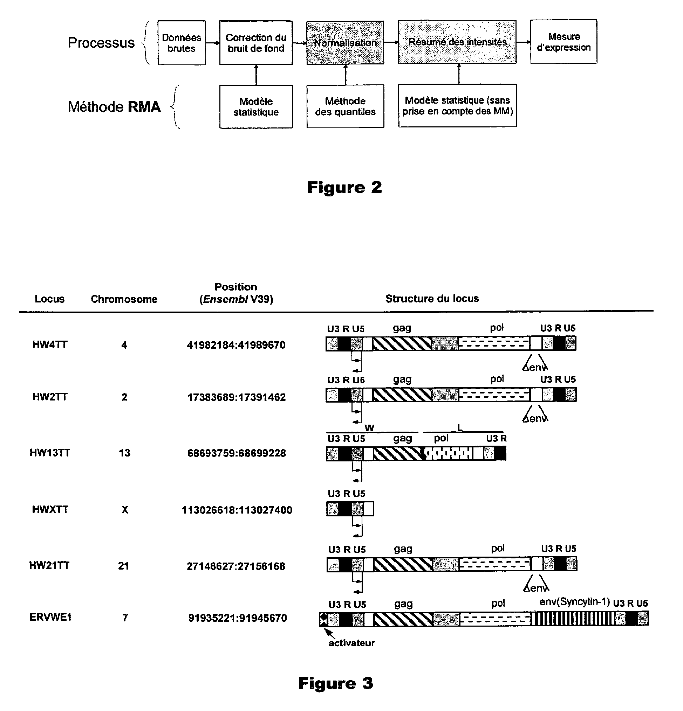

Pour chaque échantillon, 16 µg de produits d'amplification marqués par la biotine ont été hybridés sur puce à ADN selon le protocole recommandé par la société Affymetrix. Les puces ont ensuite été lavées et marquées, selon le protocole recommandé. Les puces ont enfin été lues par un scanner afin d'acquérir l'image de leur fluorescence. L'analyse d'image réalisée à l'aide du logiciel GCOS permet d'obtenir des valeurs numériques d'intensité de fluorescence qui sont prétraitées selon la méthode RMA (cf. : Figure 2) avant de pouvoir réaliser une analyse statistique pour identifier les loci HERV exprimés spécifiquement dans certains échantillons.

La comparaison des moyennes de plus de deux classes d'échantillons a été réalisée par la procédure SAM appliquée à un test de Fisher.

### Résultats :

Le traitement des données générées par l'analyse sur puce à ADN à l'aide de cette méthode a permis d'identifier six jeux de sondes correspondant à une surexpression dans un seul échantillon : le testicule tumoral. Ces cinq jeux de sondes sont spécifiques de six loci précis référencés HW4TT, HW2TT, HW13TT, HWXTT, HW21TT et ERVWE1 (cf. : Figure 3). Les informations relatives aux loci précités sont synthétisées dans le tableau 1 ci-dessous.

**Tableau 1**

| **Locus** | **SEQ ID NO :** | **Chromosome** | **Position*** |
|---|---|---|---|
| HW4TT | 8 | 4 | 41982184:41989670 |
| HW2TT | 9 | 2 | 17383689:17391462 |
| HW13TT | 10 | 13 | 68693759:68699228 |
| HWXTT | 11 | X | 113026618:113027400 |
| HW21TT | 12 | 21 | 27148627:27156168 |
| ERVWE1 | 13 | 7 | 91935221:91945670 |

| | | | |
|---|---|---|---|
| * Position donnée par rapport à la version ensemble n° 39 (juin 2006) (NCBI n° 36) http : //www.ensembl.org/Homo_sapiens/index.html | | | |

Le locus HW13TT est un provirus chimère de type HERV-W/L résultant de la recombinaison d'un provirus HERV-W et d'un provirus HERV-L. Cette chimère est telle que la région 5' constituée de la séquence partant du début de la LTR5' jusqu'à la fin du fragment gag déterminé est de type W et la région 3' constituée de la séquence partant du fragment subséquent *pol* jusqu'à la fin de la LTR3' (U3-R uniquement) est de type L. Il en résulte une fusion des régions 3'gag W-5' pol L.

### Exemple 2: Validation des loci surexprimés dans le testicule tumoral et détermination du facteur d'induction associé.

### Principe :

Les loci identifiés comme surexprimés dans le testicule tumoral à l'aide de la puce HERV haute densité ont été validés par RT-PCR en temps réel sur trois paires d'échantillons testiculaires. La spécificité de cette surexpression est évaluée par l'analyse d'échantillons provenant d'autres tissus. A cette fin, des systèmes d'amplification spécifiques ont été mis au point et utilisés pour les loci identifiés, comme décrit dans le tableau 2 ci-dessous.

**Tableau 2**

| **Locus** | **Amorce sens (SEQ ID NO :)** | **Amorce antisens (SEQ ID NO :)** |
|---|---|---|
| Gène G6PD | TGCAGATGCTGTGTCTGG (14) | CGTACTGGCCCAGGACC (15) |
| HW4TT | GGTTCGTGCTAATTGAGCTG (16) | ATGGTGGCAAGCTTCTTGTT (17) |
| HW2TT | TGAGCTTTCCCTCACTGTCC (18) | TGTTCGGCTTGATTAGGATG (19) |
| HW13TT | CATGGCCCAATATTCCATTC (20) | GGTCCTTGTTCACAGAACTCC (21) |
| HWXTT | CCGCTCCTGATTGGACTAAA (22) | CGTGGGTCAAGGAAGAGAAC (23) |
| HW21TT | ATGACCCGCAGCTTCTAACAG (24) | CTCCGCTCACAGAGCTCCTA (25) |

L'expression de ces loci est normalisée par celle d'un gène de ménage approprié : G6PD. Cette quantification d'expression a été réalisée à l'aide d'un appareil de RT-PCR en temps réel Mx3005P, commercialisé par la société Stratagene.

### Résultats :

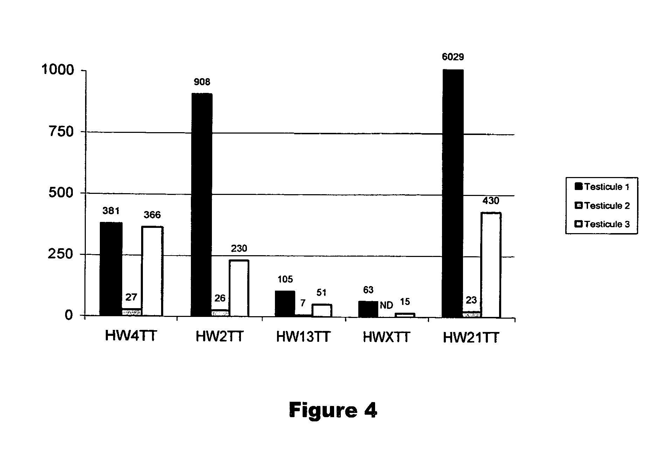

L'étude des trois paires d'échantillons testiculaires indique que tous les loci putatifs identifiés, à l'exception de HWXTT, dont l'expression n'a pas pu être quantifiée dans le second couple d'ARN testiculaire, sont surexprimés dans le testicule tumoral par rapport au tissu sain (cf. : Figure 4).

L'analyse de paires d'échantillons provenant d'autres tissus (colon, utérus, sein ovaire, poumon et prostate) montre que le phénomène de surexpression est restreint au testicule tumoral. Par conséquent, l'expression des cinq loci identifiés revêt le caractère de marqueur spécifique du cancer du testicule.

### Exemple 3 : Contrôle épigénétique de la transcription.

### Principe :

La méthylation de l'ADN est une modification épigénétique, qui s'opère, chez les eucaryotes, par l'addition d'un groupement méthyle sur les cytosines des di-nucléotides 5'-CpG, et se traduit par une répression transcriptionnelle quand cette modification a lieu au sein de la séquence nucléotidique d'un promoteur. Mises à part quelques exceptions, les séquences endogènes humaines d'origine rétrovirale sont contraintes, par ce processus de méthylation, à un état transcriptionnel silencieux dans les cellules de l'organisme en condition physiologique.

Afin d'analyser le statut de méthylation de la LTR unique ou de la LTR 5' des cinq loci, la méthode dite de « Bisulfite Sequencing PCR » a été utilisée. Cette méthode permet, à partir du séquençage d'un échantillon représentatif de la population, d'identifier l'état de méthylation de chaque dinucléotide CG sur chacune des séquences au sein du tissu étudié.

L'information de méthylation étant perdue lors des étapes d'amplification, il convient, par la méthode de traitement de l'ADN génomique par le bisulfite de sodium, de traduire l'information de méthylation au sein même de la séquence nucléotidique. L'action du bisulfite (sulfonation), suivie d'une désamination hydrolytique puis d'une désulfonation alcaline, permet en effet de modifier toutes les cytosines contenues dans l'ADN génomique en uracile. La vitesse de désamination des cytosines (C) sulfonées est cependant bien supérieure à celle des 5-méthyl-C sulfonées. On peut donc, en limitant le temps de réaction à 16 heures, transformer de manière stricte les cytosines non méthylées en uracile (U), tout en préservant les cytosines possédant un groupement méthyle. Après le traitement au bisulfite de sodium, la séquence d'intérêt est amplifiée à partir de l'ADN génomique issu de la section testiculaire tumorale et de celui issu de la section testiculaire saine adjacente par réaction de polymérisation en chaîne (PCR) en deux étapes. La première PCR permet une sélection spécifique de la séquence d'intérêt, la seconde PCR « nichée » permet d'amplifier cette séquence.

La séquence ADN ayant été modifiée par le bisulfite, la conception des amorces a tenu compte du changement de code (C en U), et les amorces ont été choisies de manière à s'hybrider sur une zone ne contenant aucun CpG (leur état de méthylation, donc de conversion, étant a priori inconnu).

Les séquences des amorces utilisées sont décrites dans les tableaux 3 à 8, ci-dessous.

**Tableau 3 - locus HW4TT**

| **Amplification** | **Amorce sens 5'→3' (SEQ ID NO :)** | **Amorce antisens 5'→3' (SEQ ID NO:)** |
|---|---|---|
| Première PCR | CCAACATCACTAACACAACCT (26) | GGGAGTTAGTAAGGGGTTTG (27) |
| PCR nichée | CAACCTATTAAACAAAACTAAATT (28) | AGATTTAATAGAGTGAAAATAGAGTTT (29) |

**Tableau 4 - locus HW2TT**

| **Amplification** | **Amorce sens 5'→3' (SEQ ID NO :)** | **Amorce antisens 5'→3' (SEQ ID NO :)** |
|---|---|---|
| Première PCR | TTATTAGTTTAGGGGATAGTTG (30) | ACACAATAAACAACCTACTAAAT (31) |
| PCR nichée | GAGGGTAAGTGGTGATAAA (32) | AACCTACTAAATCCAAAAAAA (33) |

**Tableau 5 - locus HW13TT**

| **Amplification** | **Amorce sens 5'→3' (SEQ ID NO :)** | **Amorce antisens 5'→3' (SEQ ID NO :)** |
|---|---|---|
| Première PCR | TAGGATTTTAGGTTTATTGTTA (34) | AAAAATAAAATATTAAACC (35) |
| PCR nichée | ATATGTGGGAGTGAGAGATA (36) | CAACAACAAACAATAATAATAA (37) |

**Tableau 6 - locus HWXTT**

| **Amplification** | **Amorce sens 5'→3' (SEQ ID NO :)** | **Amorce antisens 5'→3' (SEQ ID NO:)** |
|---|---|---|
| Première PCR | TTGAGTTTTTTTATTGATAGTG (38) | TCTAAATCCTATTTTCCTACT (39) |
| PCR nichée | GTTTTTTTATTGATAGTGAGAGAT (40) | TAACAAACCTTTAATCCAAT (41) |

**Tableau 7 - locus HW21TT**

| **Amplification** | **Amorce sens 5'→3' (SEQ ID NO :)** | **Amorce antisens 5'→3' (SEQ ID NO :)** |
|---|---|---|
| Première PCR | TTTAGTGAGGATGATGTAATAT (42) | CAACTTAATAAAAATAAACCCA (43) |
| PCR nichée | ATAATGTTTTAGTAAGTGTTGGAT (44) | ACAATTACAAACCTTTAACC (45) |

**Tableau 8 - locus ERVWE1**

| **Amplification** | **Amorce sens 5'→3' (SEQ ID NO :)** | **Amorce antisens 5'→3' (SEQ ID NO :)** |
|---|---|---|
| Première PCR | AATTCATTCAACATCCATTC (46) | GGTTTAATATTATTTATTATTTTGGA (47) |
| PCR nichée | CTCTTACCTTCCTATACTCTCTAAA (48) | AGAGTGTAGTTGTAAGATTTAATAGAGT(49) |

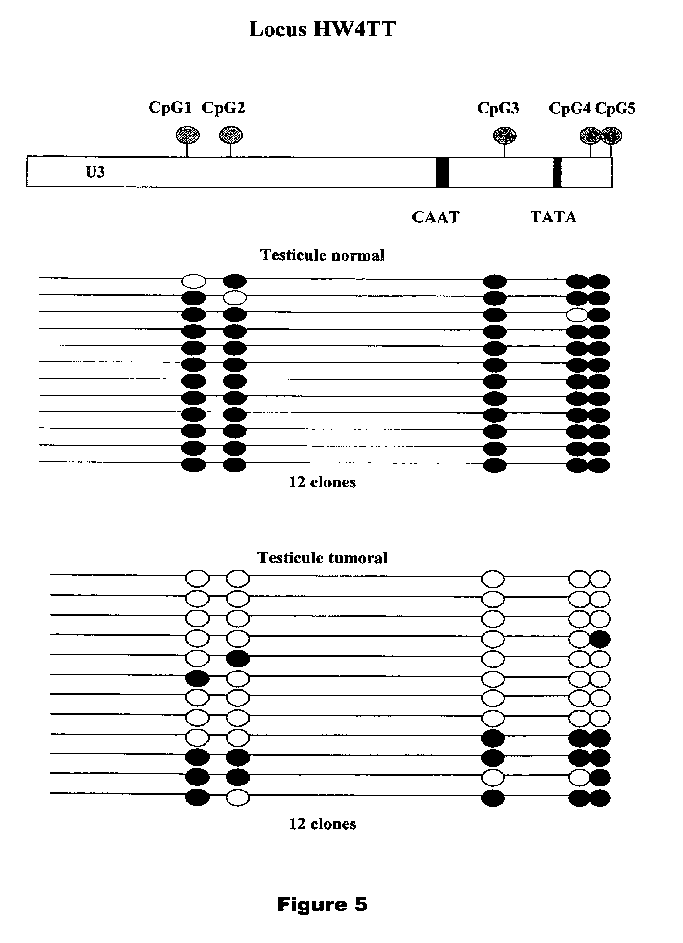

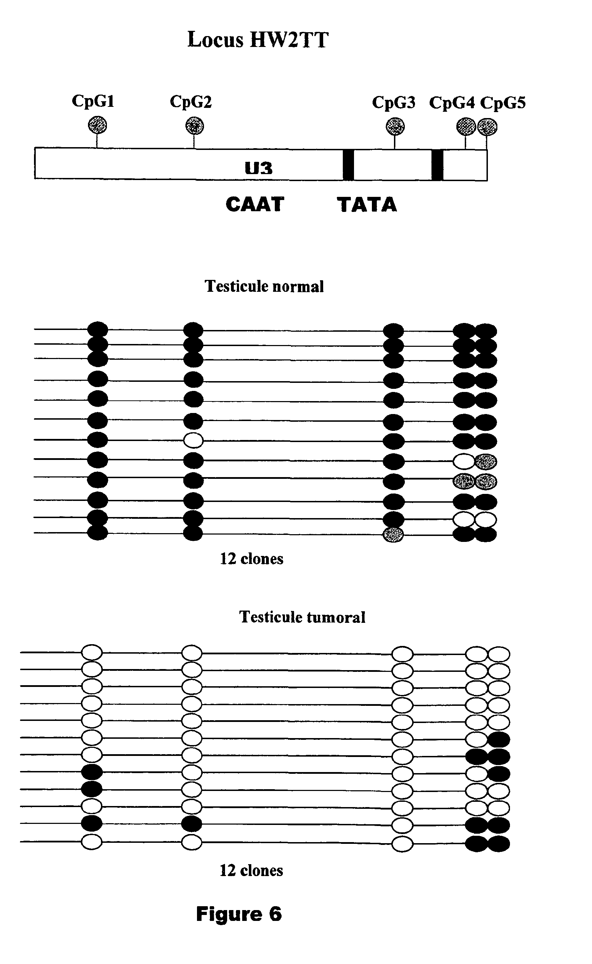

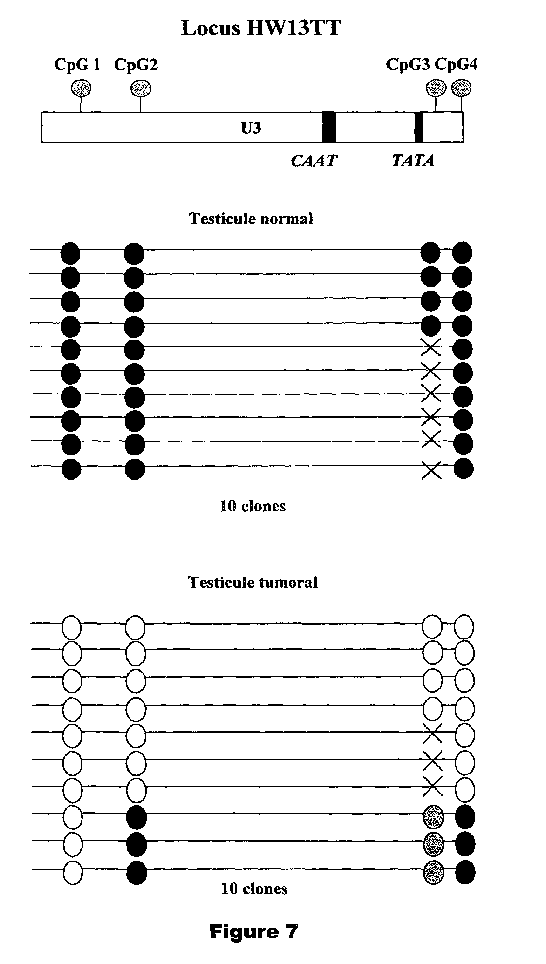

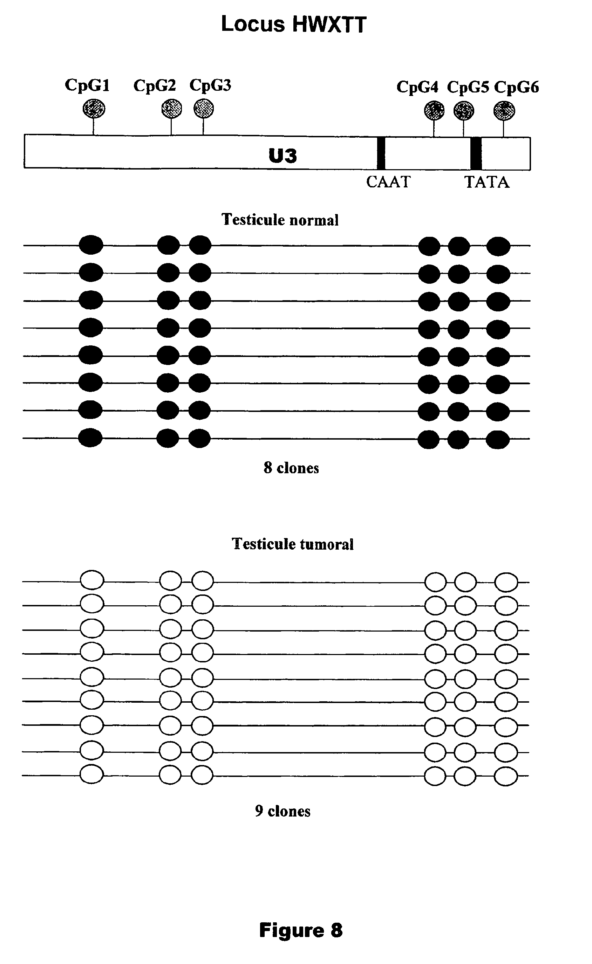

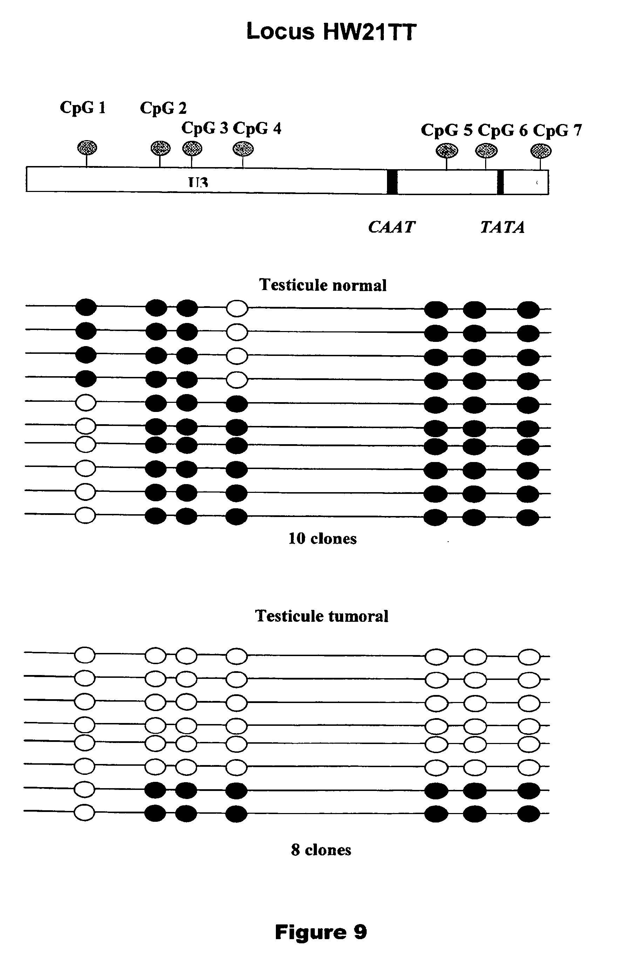

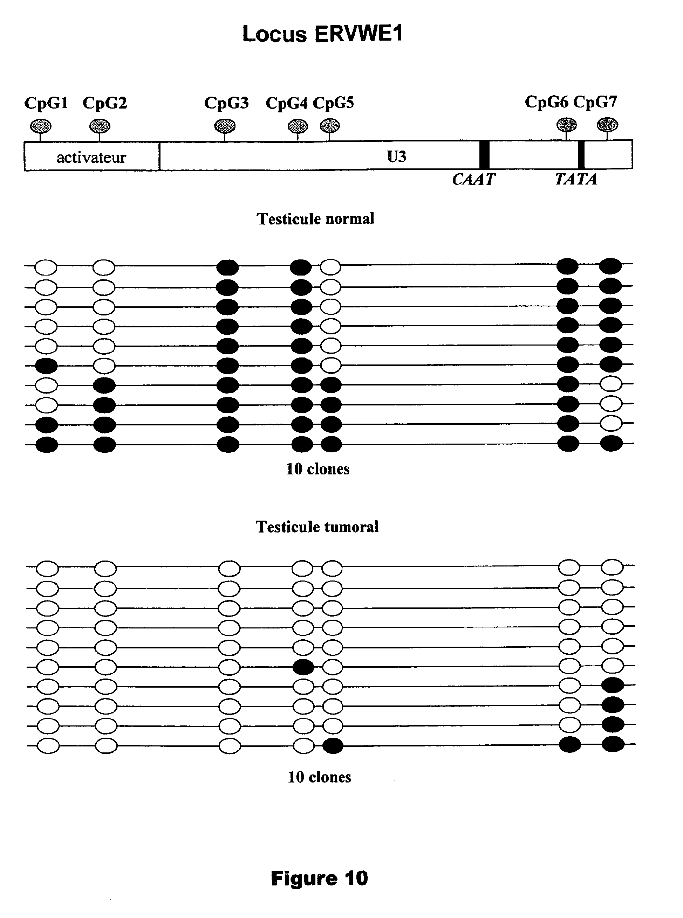

Après extraction sur gel, et purification, les amplicons sont clonés dans des plasmides, et ces derniers sont utilisés pour transformer des bactéries compétentes. Une douzaine de minipréparations d'ADN plasmidique sont réalisées à partir des bactéries transformées et les amplicons contenus dans les plasmides sont séquencés. On procède alors à l'analyse des séquences obtenues (cf. : Figures 5 à 10).

### Résultats :

L'analyse de la région 5' des transcrits des loci identifiés a été réalisée à l'aide de la technique 5' Race. Elle a notamment permis de montrer que la transcription est initiée au début de la région R de la LTR 5' provirale. Ceci traduit l'existence d'un rôle promoteur de la région U3 de la LTR 5' provirale.

### 1. Etat de méthylation des séquences U3 de la LTR5'du locus HW4TT :

La séquence U3 de la LTR5' du locus HW4TT de référence contient 5 sites CpGs.
a) dans l'échantillon de tissu testiculaire sain : sur 12 séquences analysées, 9 sont totalement méthylées. Les 3 autres présentent à chaque fois 1 CpG non méthylé sur les 5 que compte la région U3. Ceci représente donc une méthylation globale de la région U3 de la LTR5' du locus HW4TT s'élevant à 95% dans l'échantillon testiculaire sain ;
b) dans l'échantillon de tissu testiculaire tumoral : sur 12 séquences analysées, 5 (soit 41,66% des séquences) sont totalement déméthylées, 3 séquences ont 4 CpG sur 5 non méthylés, 2 séquences ont 2 CpG sur 5 non méthylés, 1 séquence a 1 CpG sur 5 non méthylé, et 1 séquence reste totalement méthylée. Ceci représente donc une méthylation globale de la région U3 de la LTR5' du locus HW4TT s'élevant à 30% dans l'échantillon testiculaire tumoral.

### 2. Etat de méthylation des séquences U3 de la LTR5'du locus HW2TT :

La séquence U3 de la LTR5' du locus HW2TT de référence contient 5 sites CpGs.
a) dans l'échantillon de tissu testiculaire sain : sur 12 séquences analysées, 9 sont totalement méthylées, 1 possède son 2^{ème} CpG non méthylé, 1 a le CpG en position 4 non méthylé, 1 a les CpG en position 4 et 5 non méthylés et 3 séquences présentent des mutations ponctuelles sur un ou deux CpG (une en position 3, une en position 5 et une en positions 4 et 5), reflétant très probablement des artefacts de PCR. Ceci représente donc une méthylation globale de la région U3 de la LTR5' du locus HW2TT s'élevant à 92,9% dans l'échantillon testiculaire sain ;
b) dans l'échantillon de tissu testiculaire tumoral : sur 12 séquences analysées, 6 sont totalement déméthylées. 5 séquences possèdent un ou deux CpG méthylé(s) (1 à la position 1, 1 autre à la position 5, 1 sur les positions 1 et 5, 2 aux positions 4 et 5 et 1 à la position 3). Enfin, une séquences possède 4 CpG méthylés sur 5 (positions 1, 2, 4 et 5). Ceci correspond à une méthylation globale de la région U3 de la LTR5' du locus HW2TT s'élevant à 20% dans l'échantillon testiculaire tumoral.

### 3. Etat de méthylation des séquences U3 de la LTR5'du locus HW13TT :

La séquence U3 de la LTR5' du locus HW 13TT de référence contient 3 sites CpGs.
a) dans l'échantillon de tissu testiculaire sain : un CpG supplémentaire par rapport à la séquence de référence est retrouvé dans 4 des 10 clones étudiés pour ce locus. Il est situé entre les CpG 2 et 3 et est méthylé. Dans les 6 autres clones, ce site est muté par rapport à la séquence de référence. Les 3 autres CpG de la région U3 sont méthylés dans les 10 séquences analysées. Ceci représente donc une méthylation globale de la région U3 de la LTR5' du locus HW13TT s'élevant à 100% dans l'échantillon testiculaire sain ;
b) dans l'échantillon de tissu testiculaire tumoral : le CpG supplémentaire indiqué ci-dessus est également retrouvé. Il est déméthylé dans 4 des 10 séquences analysées, muté dans 3 autres séquences et son état de méthylation est indéterminé dans les 3 dernières séquences. 7 séquences sur 10 sont totalement déméthylées et les 3 autres sont méthylées sur le 2^{ème} et sur le 3^{ème} CpG. Ceci correspond à une méthylation globale de la région U3 de la LTR5' du locus HW 13TT s'élevant à 20% dans l'échantillon testiculaire tumoral.

### 4. Etat de méthylation des séquences U3 de la LTR solitaire du locus HWXTT :

La séquence U3 de la LTR5' du locus HWXTT de référence contient 6 sites CpGs.
a) dans l'échantillon de tissu testiculaire sain : les 8 séquences analysées sont totalement méthylées, ce qui correspond à un pourcentage de méthylation de 100% dans l'échantillon testiculaire sain ;
b) dans l'échantillon de tissu testiculaire tumoral : les 9 séquences analysées 6 sont totalement déméthylées, ce qui correspond à un pourcentage de méthylation de 0%.

### 5. Etat de méthylation des séquences U3 de la LTR5'du locus HW21TT :

La séquence U3 de la LTR5' du locus HW21TT de référence contient 7 sites CpGs.
a) dans l'échantillon de tissu testiculaire sain : les 10 séquences analysées ont toutes 6 CpG méthylés sur 7, pour 6 des séquences le 1^{er} CpG est non méthylé et pour les 4 autres séquences le 4^{ème} CpG est non méthylé. Ceci représente donc une méthylation globale de la région U3 de la LTR5' du locus HW21TT s'élevant à 85,7% dans l'échantillon testiculaire sain ;
b) dans l'échantillon de tissu testiculaire tumoral : sur 8 séquences analysées, 6 sont totalement déméthylées, 2 autres présentent un profil identique à l'un de ceux trouvés dans le tissu testiculaire sain, à savoir 6 CpG méthylés et le 1^{er} CpG non méthylé. Ceci correspond à une méthylation globale de la région U3 de la LTR5' du locus HW21TT s'élevant à 21,4% dans l'échantillon testiculaire tumoral.

### 6. Etat de méthylation des séquences de l'activateur et de la U3 de la LTR 5' du locus ERVWE1:

Le locus ERVWE1 comprend, en plus de sa région U3 promotrice, un activateur connu localisé directement en amont de la LTR5', et qui contient deux sites CpG (CpG 1 et 2). La séquence U3 de la LTR5' du locus ERVWE1 de référence contient qant à elle 5 sites CpGs (CpG 3 à 7).
a) dans l'échantillon de tissu testiculaire sain : sur 10 séquences analysées, 5 séquences ont les CpG 1 et 2 (activateur) et 5 (U3) non méthylés, 1 séquence a les CpG 2 et 5 non ùéthylés, 2 séquences ont les CpG 1 (activateur) et 7 (U3) non méthylés, 1 séquence a le CpG 7 uniquement non méthylé, et 1 enfin est totalement méthylée pour les 7 CpG. Au total, ceci correspond à un pourcentage de méthylation de 68,57% dans l'échantillon testiculaire sain ;
b) dans l'échantillon de tissu testiculaire tumoral : sur les 10 séquences analysées, seules 3 séquences présentent pour chacune un unique CpG méthylé (CpG 4 ou CpG5 ou CpG6), les 7 autres séquences sont totalement déméthylées, ce qui correspond à un pourcentage de méthylation de 4,29%.

Le niveau de méthylation très élevé des promoteurs rétroviraux U3 des loci considérés dans le tissu sain indique une répression de l'expression transcriptionnelle par un mécanisme épigénétique. En revanche, le niveau faible de méthylation de ces mêmes promoteurs dans le tissu tumoral traduit une levée d'inhibition transcriptionnelle dont découle l'expression significativement plus élevée mise en évidence à l'aide de la puce ADN HERV haute densité et à l'aide de la RT-PCR en temps réel. Ainsi, les promoteurs rétroviraux U3 des loci considérés apparaissent comme des marqueurs spécifiques du caractère tumoral du testicule.

### Références bibliographiques

[1] Nickerson D. A. et al., DNA sequence diversity in a 9.7 -kb region of the human lipoprotein lipase gene, Nature Genetics, Vol. 19, pp 233-240 (1998).
[2] Cottrell S. E., Molecular diagnostic applications of DNA methylation technology, Clinical Biochemistry 37, pp 595-604 (2004).

## Revendications

1. Procédé pour le diagnostic ou le pronostic, *in vitro,* du cancer du testicule dans un échantillon biologique issu d'un patient suspecté d'être atteint d'un cancer du testicule, **caractérisé en ce qu'**il comprend une étape de détection du niveau de
qu'il étape de détection de
étape de détection de méthylation des dinucléotides CpG dans au moins une séquence cible d'ADN génomique de l'échantillon, la séquence cible étant choisie parmi au moins une des séquences identifiées en SEQ ID NOs : 1 à 7 ou parmi au moins une séquence qui présente au moins 99% d'identité avec une des séquences identifiées en SEQ ID NOs : 1 à 7 et leurs séquences complémentaires.

2. Procédé selon la revendication 1, **caractérisé en ce qu'**il comprend :
(i) une extraction de l'ADN génomique à analyser dans l'échantillon,
(ii) un traitement de l'ADN génomique extrait avec un ou plusieurs réactifs pour convertir les bases cytosines, des dinucléotides CpG, non méthylées en position 5, en uraciles,
(iii) au moins une amplification de l'ADN traité,
(iv) la détermination, sur la base de la présence ou de l'absence de méthylation des dinucléotides CpG, d'un statut de méthylation de ladite séquence cible ou d'une valeur qui reflète un état de méthylation de la séquence cible.

3. Procédé selon la revendication 2, **caractérisé en ce que** dans (ii) le traitement de l'ADN génomique comprend l'utilisation d'une solution choisie dans le groupe consistant en bisulfite, disulfite, sulfite d'hydrogène et leurs combinaisons.

4. Procédé selon l'une quelconque des revendications précédentes, **caractérisé en ce que** l'échantillon est choisi parmi un extrait tissulaire de testicule ou un fluide biologique, tel que le sang, le sérum, le plasma, l'urine et le liquide séminal.

5. Séquence d'acide nucléique, isolée, **caractérisée en ce qu'**elle consiste en au moins une séquence ADN choisie parmi les séquences identifiées en SEQ ID NOs : 1 à 7 ou parmi au moins une séquence qui présente au moins 99% d'identité avec une des séquences identifiées en SEQ ID NOs : 1 à 7 ou leurs séquences complémentaires.

6. Utilisation d'au moins une séquence telle que définie dans la revendication 5, comme marqueur pour le diagnostic ou le pronostic *in vitro* du cancer du testicule.

## Patentansprüche

1. Ein Verfahren zur Diagnose oder Prognose, *in vitro,* von Hodenkrebs in einer biologischen Probe eines Patienten, von dem angenommen wird, dass er an Hodenkrebs erkrankt ist, **dadurch gekennzeichnet, dass** es einen Schritt zum Erfassen des Grades an Methylierung der CpG-Dinukleotiden in mindestens einer Target-Sequenz von genomischer DNA der Probe beinhaltet, wobei die Target-Sequenz aus mindestens einer der in SEQ ID NO: 1 bis 7 identifizierten Sequenzen oder aus mindestens einer Sequenz, die zu mindestens 99 % mit einer der in SEQ ID NO: 1 bis 7 identifizierten Sequenzen und deren Komplementärsequenzen identisch ist, ausgewählt ist.

2. Verfahren gemäß Anspruch 1, **dadurch gekennzeichnet, dass** es Folgendes beinhaltet:
(i) Extrahieren der zu analysierenden genomischen DNA in der Probe,
(ii) Behandeln der extrahierten genomischen DNA mit einem oder mehreren Reaktanten, um die Cytosinbasen, der CpG-Dinukleotide, die bei Position 5 nicht methyliert sind, in Uracile umzuwandeln,
(iii) mindestens ein Amplifizieren der behandelten DNA,
(iv) Bestimmen, basierend auf der Anwesenheit oder Abwesenheit von Methylierung der CpG-Dinukleotide, eines Methylierungsstatus der Target-Sequenz oder eines Wertes, der einen Methylierungszustand der Target-Sequenz widerspiegelt.

3. Verfahren gemäß Anspruch 2, **dadurch gekennzeichnet, dass** in (ii) das Behandeln der genomischen DNA das Verwenden einer Lösung beinhaltet, die ausgewählt ist aus der Gruppe, bestehend aus Bisulfit, Disulfit, Wasserstoffsulfit und Kombinationen davon.

4. Verfahren gemäß einem der vorhergehenden Ansprüche, **dadurch gekennzeichnet, dass** die Probe aus einem Hodengewebeauszug oder einem biologischen Fluid wie etwa Blut, Serum, Plasma, Urin oder Samenflüssigkeit ausgewählt ist.

5. Eine Nukleinsäuresequenz, die isoliert ist, **dadurch gekennzeichnet, dass** sie aus mindestens einer DNA-Sequenz besteht, die ausgewählt ist aus den in SEQ ID NO: 1 bis 7 identifizierten Sequenzen oder aus mindestens einer Sequenz, die zu mindestens 99 % mit einer der in SEQ ID NO: 1 bis 7 identifizierten Sequenzen oder deren Komplementärsequenzen identisch ist.

6. Eine Verwendung mindestens einer Sequenz gemäß Anspruch 5 als Marker zur Diagnose oder Prognose von Hodenkrebs *in vitro.*

## Claims

1. A method for *in vitro* diagnosis or prognosis of testicular cancer, in a biological sample from a patient suspected of suffering from testicular cancer, **characterized in that** it comprises a step of detecting the level of methylation of CpG dinucleotides in at least one genomic DNA target sequence of the sample, the target sequence being selected from at least one of the sequences identified in SEQ ID Nos.: 1 to 7 or from at least one sequence which exhibits at least 99% identity with one of the sequences identified in SEQ ID Nos.: 1 to 7 and the sequences complementary thereto.

2. The method as claimed in claim 1, **characterized in that** it comprises:
(i) extraction of the genomic DNA to be analyzed from the sample,
(ii) treatment of the extracted genomic DNA with one or more reagents so as to convert the cytosine bases, of the CpG dinucleotides, which are nonmethylated at position 5, into uracils,
(iii) at least one amplification of the treated DNA,
(iv) determination, on the basis of the presence or absence of methylation of the CpG dinucleotides, of a methylation status of said target sequence or of a value which reflects a methylation state of the target sequence.

3. The method as claimed in claim 2, **characterized in that**, in (ii), the treatment of the genomic DNA comprises the use of a solution selected from the group consisting of hydrogen. sulfite, disulfite and bisulfite, and combinations thereof.

4. The method as claimed in any one of the preceding claims, **characterized in that** the sample is selected from a testicular tissue extract or a biological fluid, such as blood, serum, plasma, urine and seminal fluid.

5. An isolated nucleic acid sequence, **characterized in that** it consists of at least one DNA sequence selected from the sequences identified in SEQ ID Nos. 1 to 7 or from at least one sequence which exhibits at least 99% identity with one of the sequences identified in SEQ ID Nos. 1 to 7 or the sequences complementary thereto.

6. The use of at least one sequence as defined in claim 5, as a marker *for in vitro* diagnosis or prognosis of testicular cancer.


### SEQUENCE LISTING

<110> bioMérieux
<120> Procédé pour le diagnostic ou pronostic in vitro du cancer du testicule
<130> Cancerendo2PCT
<150> FR0851621
   <151> 2008-03-12
<160> 49
<170> PatentIn version 3.3
<210> 1
   <211> 255
   <212> DNA
   <213> Homo sapiens
<400> 1
   tgagaaacag gactagttag atttcctagg ccaactaaga atccctaagc ctagctggga      60
   aggtgatcgc atccaccttt aaacacgggc ttgcaactta gctcacacct gaccaatcag     120
   gtagtaaaga gagctcacta aaatgctaat taggcaaaaa caggaggtaa agaaatagcc     180
   aatcatctat tgcctgacac cacacgggga gggacaatga ttgggatata aacccaggaa     240
   ttcgagctgg caacg                                                      255
<210> 2
   <211> 247
   <212> DNA
   <213> Homo sapiens
<400> 2
   tgagacacag gactagctgg atttcctagg ccgactaaga atccctaagc ctagctggga      60
   aggtgaccac atccaccttt aaacacgggg tttacaactt agctcacacc cagccaatca     120
   gagagctcac taaaatgcta attaggcaaa aacaggaggt aaagaaatag ccaatcatct     180
   attgcctgag agcacagcgg gagggacaag gattgggata taaacccagg cattcgagct     240
   ggcaacg                                                               247
<210> 3
   <211> 241
   <212> DNA
   <213> Homo sapiens
<400> 3
   tgagagacag ctggatttcc taggccgact aagaatccct aagcctagct gggaaggtga      60
   ccgcatccac ctttaaacac agggcttgca acttagctca cacccaacca atcagagagc     120
   tcactaaaat gctaattagg caaaaacagg aggtaaagaa atagcaagtc atctattgcc     180
   tgagagcaca gtgggaggga caaggaccag gatataaacc caggcatttg agccagcaac     240
   g                                                                     241
<210> 4
   <211> 248
   <212> DNA
   <213> Homo sapiens
<400> 4
   tgagagacag gactaactgg atttcctagg ccgactaaga atccctaagc ctagctggga      60
   aggtgaccgc atccatcttt aaacacgggg cttgaaactt agctcacacc taaccagtca     120
   gagagctcac taaaatgcta attaggcaaa aaacaggagg taaagaaata gccaatcatc     180
   tattgcctga gagcacagcg ggagggacaa ggatcgggat ataaacccag gcattcgagc     240
   cagcaatg                                                              248
<210> 5
   <211> 255
   <212> DNA
   <213> Homo sapiens
<400> 5
   tgagagacag gactagctgg atttcctagg ccgactaaga atccctaagc ctagctggga      60
   aggtgaccgc ttccaccttt aaacacgggg cttgcaactt agctcacacc cgaccaatca     120
   gatagtaaag agagcacact aaaatgctaa ttaggcaaaa acaggaggta aagaaatagc     180
   caatcatcta ttgcctgaga gcaaagcggg agggacaatg atcgggatag aaacccaggc     240
   attcaagccg gaatg                                                      255
<210> 6
   <211> 247
   <212> DNA
   <213> Homo sapiens
<400> 6
   tgagagacag gactagctgg atttcctagg ccgactaaga atccctaagc ctagctggga      60
   aggtgaccac gtccaccttt aaacacgggg cttgcaactt agctcacacc tgaccaatca     120
   gagagctcac taaaatgcta attaggcaaa gacaggaggt aaagaaatag ccaatcatct     180
   attgcctgag agcacagcag gagggacaac aatcgggata taaacccagg cattcgagct     240
   ggcaaca                                                               247
<210> 7
   <211> 314
   <212> DNA
   <213> Homo sapiens
<400> 7
   ccctggggcg ggcttccttt ctgggatgag ggcaaaacgc ctggagatac agcaattatc      60
   ttgcaactga gagacaggac tagctggatt tcctaggccg actaagaatc cctaagccta     120
   gctgggaagg tgaccacgtc cacctttaaa cacggggctt gcaacttagc tcacacctga     180
   ccaatcagag agctcactaa aatgctaatt aggcaaagac aggaggtaaa gaaatagcca     240
   atcatctatt gcctgagagc acagcaggag ggacaacaat cgggatataa acccaggcat     300
   tcgagctggc aaca                                                       314
<210> 8
   <211> 7774
   <212> DNA
   <213> Homo sapiens
<400> 8
   tgagacacag gactagctgg atttcctagg ccgactaaga atccctaagc ctagctggga      60
   aggtgaccac atccaccttt aaacacgggg tttacaactt agctcacacc cagccaatca     120
   gagagctcac taaaatgcta attaggcaaa aacaggaggt aaagaaatag ccaatcatct     180
   attgcctgag agcacagcgg gagggacaag gattgggata taaacccagg cattcgagct     240
   ggcaacggca accccctttg ggtctcctcc ctttgcatag gagctctgtt ttcactctat      300
   taagtcttgc aactgcactc ttctggtccg tgtttcttac cgcttgagct gagctttccc     360
   tcactgtcca ccactgctgt tttgccaccg tcacaggccc accgctgact tccattcttc     420
   tggatctagc aggctgtcca ctgtgctcct gatccagcga ggcgcccatt gccgctcccg     480
   attgggctaa aggcttgcca ttgttcctgc atggctacgt gcctgggttc atcctaatca     540
   agccgaacac tagtcactgg gttccacggt tctcttccat gacccacgac ttctaataga     600
   actataacac tcacctcatg gcccaagatt ccattccttg gaatccatga ggccaagaac     660
   cccaggtcag agaacacgag gcttgccacc atcttggaag tggccccacc accatcttgg     720
   gagctctggg agcaaggacc cccggtaaca ttttggcgac cacaaaggga catccaaagt     780
   ggtgagtaat attggaccac tttcacttgc tattctgttc tatccttcct tagaactgga     840
   ggaaaatacc aggcacaggc acctgtcagc cagttaaaaa caattagcgt cgccgccaca     900
   cttaagactc aggtgtgagg ctatctgggg aaagactttc taacaacccc caacccatct     960
   agtggggatg ttggtctgcc tggagacagc ttccactttc aattttcttg gggaagccga    1020
   gggctcacta gaggcagaca gctgttgtcc caaactccgg gcagtagccg gttgagatca    1080
   tggtgcagcc aggagtctct actcagcagt cgccgatgca tgtgccccta ccttcccttc    1140
   tgacccatac atcctgagtc ccgactgtga ctttcttgaa agtgtagccc caaaattctc    1200
   cttacctctg aatctacttc ctctgatccc tgcctcctgg gtactaatga ttcagacttt    1260
   catttcctct agcaagttgt gtctccaaag ggatctaagg aggctctacg ctgcatcctt    1320
   aggcacctag gctataaccc aaggagtctt atccctggtg tccctcccga tttgggtata    1380
   caactctcaa catgggcagt tatgtaggac ccattcccca ccacacttgc cagggcccca    1440
   agtttgtaat ggctaagaga gagacacaga gagagagaga gagatggaga gagagacaag    1500
   gagggagtca aagagaaaaa gaaagaaaaa gaaatagtag aaaaaaaagt gtgccctatt    1560
   cctttaaaag ccagggtaaa tttaaaacct gtaattgata attgaaggtc ttctccgtga 1620
   ccctgtaaca ctccaatgcc attttgttgt cagtgtaaat aagggcatag cccaaaagca 1680
   ctgaggtcac tgacaacccg tagctttccc atcaaaaatc cttaacccag taatccgcgg 1740
   atgggccaaa tgcattcagt cggtagcagc aaccgctttg ctaaaagtag aaaagtaact 1800
   tttagaggaa acctcattgt gagcgcacac ctcaccagtt cagaattatt ctaagtcaaa 1860
   aaaaaaaaaa gcaaaaaggt aacttactaa ctcaaaaatc ttaaagtata ggtctatcat 1920
   attagaaaag ggtaatgtaa ctccaaccac tgataattcc cttaacccag cagatttcct 1980
   aacaggggat ttaaaactta attaccatac aaaggtccca ccagacctag gaggaactcc 2040
   cttcaggaca ggacgataaa cggttcctcc caggtgattg aggaaaaaaa ccacaatggg 2100
   tattcagtaa ttgatacaga gactcatgtg gaagcagtta gaaaaattgc ctaataattg 2160
   gtctcctcaa acgtgtaagc tgtttgcact cagccaagcc ttaaagtact tacagaatca 2220
   aaaagactct gaatcctgac tcaaaaggtt tgctacaccc tctgtgaaac aaatttgcat 2280
   aagaactgtt gtttatggga aggcatcttg atggggcagc tgggttgtta tgaaatactc 2340
   aggaccccag cccggctcta ggactcaccc ctgagcgcaa aaggcaatgt tgggcacgct 2400
   ggtaaaggac cactagaatc cagcagcccg gacccctttc tttgtggtca agagaggcgg 2460
   gaaaacaggt gcaggactgc tacatcagtg agcataacta atccagtaag cagaggtcca 2520
   tgggtggtta tgcaccctgg aaaagaatac gcattaggcc cttagaggat gctctaggac 2580
   taatgctcat cggaaaatga ctaggggtgc tgacatccct atgttctttt ttcagatggg 2640
   aaacgttcct cccaccccaa ggcaaaaaac acccctaaga tgtattttgg agaattagga 2700
   ccaatttgac cctcagacac taagaaagaa atgacttaca ttcttctgca gtaccatgat 2760
   atcctcttca agggggagaa acctggcctc ctgagagaag tataaattat aacaccatct 2820
   tacagtgaga cctcttctgt agaaaggagg gcaaatggag tgaagtgcaa actttccttt 2880
   cattaagaga caactcgcaa ttatgtaaaa agtgtgattt atgccctaca gaaagccctc 2940
   agtctacctc cctatcccag ggtccccccg attcctttcc caactaataa ggacccccct 3000
   tttacccaaa tggtccaaag gagatagatg aagggataaa caatgaacca aacagtgcca 3060
   atattccctg attatgcccc ctccaggcag tgggaggagg agaattcggc ccagccagag 3120
   tgcatgtacc tttttttttc tctcagactt aaagcaaatt aaaatagacc taggtaaatt 3180
   ctcagataac cctgatggct atattgatgt tttacaaggg ttaggacaat cctttgctct 3240
   gacatggaga gatataatgt tactgctaaa tcagacacta accccaaatg agagaagtgt 3300
   caccatagct gcagcccaag agtttggcaa tctctggtat ctcagtcagg tcaatgatag 3360
   gatgacaaca gaggaaaggg aatgattccc cacaggccag caggcagttc tcagtgtaga 3420
   ccctcactgg gacacagaat aagaacatgg agatcggtgc cgcagatatt tgctaacttg 3480
   cgtgctagga ctaaggaaaa ctaggaagaa gcctatgaat tattcagtga tgtccactat 3540
   aacacaggga aaggaagaaa atcatactgc ctttccggaa atactaaggg aggcattgag 3600
   gaagcatacc tctctgtcac ctgactgtat tgaagtccaa ctaatcttaa aggatatgtt 3660
   tatcactcag tcagctgcag acattagaaa aaacttcaaa agtccacctt aggcccagag 3720
   caaaacttag aaaccctatt gaacttgtta acctcagttt tttataatag agatcaggag 3780
   gagcaggcgg aacaggacaa acaggattaa aaaaagacca ccgctttagt catggccctc 3840
   aggcaagtgg actttggaag ctctggaaaa gggaaaagct gggcaaattg aatgcctaat 3900
   agggcttgct tccagtgtgg tctacaagga cacttaaaaa aagattgtcc aagtagaaat 3960
   aagctgcccc ttcgtccatg cctcttatgt caagggaatc actggaaggc ccattgcccc 4020
   aggggaggaa ggtcctctga gtcagaagcc actaaccaga tgatccagca gcaggactaa 4080
   gggtgcccag ggcaagcccc agcccatgcc atcaccctca cagagccccg ggtatgcttg 4140
   accattgagg gccaggaggt taactgtctc ctgaacactg gcacagcctt ctcagtctta 4200
   ctttcctgtc ccggacaact gtcctccaga tctgtcacta tctgagcggt cctaggacag 4260
   ccagtcacta gatatttctc ccagccacta agttgtgact ggggaacttt actcttttca 4320
   catgcttttc taattatgcc tgaaagcccc actcctttgt tagggagaga cattctagca 4380
   aaagcagggg ccattataca tctgaacata ggagaaggaa cacccgtttg ttgtcacctg 4440
   cttgaggaag gaattaatgc tgaagtctgg gcaacagaag gacaatatgg atgagcaaag 4500
   aatgcccatc ctgttcaagt taaattaaag gattccgcct cctttcccta ccaaaggcaa 4560
   taccccctta gacccgaggc ccaacaagga ctccaaaaga ttgttaagga cctaaaagcc 4620
   caaggcctag taaaaccatg caatagcccc tgccatactc caattttagg agtaaggaaa 4680
   cccaacggac agtggaggtt agtgcaagaa ctcaggatta tcaatgaggc tgttgttcct 4740
   ctatacccag ctgtacctaa cccttataca gtgctttccc aaataccaga ggaagcagag 4800
   tggtttacag tcctggacct taaggatgcc tttttctgca tccctgtacg tcctgactct 4860
   caattcttgt ttgcctttga agatcctttg aacccaacgt ctcaactcac ctggactgtt 4920
   ttaccccaag ggttcaagga tagcccccat ctatttggcc aggcattagc ccaagacttg 4980
   agccaattct catacctgga cactcttatc cttcggtatg gggatgattt aattttagct 5040
   acccattcag aaacgttgtg ccatcaagcc acccaagtgc tcttaaattt cctcgctacc 5100
   tgtggctaca ggtttccaaa cgaaaggctc agctctgctc acagcaggtt aaatacttag 5160
   ggctaaaatt atccaaaggc accagggccc tcagtgagga acgtatccag cctatactgg 5220
   cttattctca tcccaaaacc ctaaagcaac taagagcatt ccttggcata acaggctgct 5280
   gctgaatatg gattcccagg tacagtgaaa tagccaggcc attatacaca ctaattaagg 5340
   aaactcagaa agccaatacc catttagtaa gatggacacc ttaagcagaa gcggctttcc 5400
   aggccttaaa gaaggcccta acccaagccc cagtggtaag cttgccaaca gggcaagact 5460
   tttctttata tgtcacagaa gaaacaggaa tagctctagg agtccttaca caggtctgag 5520
   ggatgagctt gcaacccatg gcatacctga gtaaggaaac tgatgtagtg gcaaagggtt 5580
   ggcctcattg tttacgggta gtggcagcag tagcagtctt agtatctgaa gtagttaaaa 5640
   taatacaggg aagagatctt actgtgtgaa catctcatga tgtgaatggc atagtcactg 5700
   ctaaaggaga cttgtggctg tcagacaact gtttacttaa ataccaggct ctattacttg 5760
   aagggccagt gctgcgactg tgcacttgtg caactcttaa cccagacaca tttcttccag 5820
   acaatgaaga aaagatagaa cataactgcc aacaagtaat tgctcaaacc tatgccactc 5880
   gaggggacct tttagaggtt cccttgactg atcccaacct caacttgtat actgatggaa 5940
   gttcctctgt agaaaaagga ctttgaaaag tggggtatgc agtggtcagt gataatggaa 6000
   tacttgaaag taatcccctc actccaggaa ctagtgctca gctggcagaa ctaatagccc 6060
   tcactcgggc actagaatta ggagaagaga aaagggtaaa tatatacaga ctctaagtat 6120
   gcttacctag tcctccatgc ccatgcagca atatggagag aaagggaatt cctaatttcc 6180
   aagggaacac ctatccaaca tcaggaagcc attaggagat tactattggc tgtacagaaa 6240
   cataaagagg tggcaatctt acactgccgg tgtcaccaga aaggaaagga aagggaaata 6300
   gaaaggaacc accaagcgga tattgaagcc aaaagagccg caaggcagga ccctccatta 6360
   gaaatgctta tagaaggacc cctagtatgg ggtaatcccc tccaggaaac caagccccag 6420
   tactcagaag aagaaataga atgaggaacc tcacaagcac atagtttcct cccctcagga 6480
   tggctagcca ctgaagaagg aaaaatactt ttgcctgcag ctaaccaatg gaaattactt 6540
   aaaacccttc accaaacatt tcccttaggc attgatagca cccatcagat ggccaaatta 6600
   ttatttactg gaccaggcct tttcaaaact atcaagcaga tagtcagggc ctgtaaagtg 6660
   tgccaaacaa gtaatcccct gcactgcagg ccatacattt caatccctgt atctttaacc 6720
   tccttgttaa gtttgtctct tccagaatca aagctgtaaa actacaaata gttcttcaaa 6780
   tggagcccca gatgtagtcc atgactaaga tctaccgcgg acccctggac aagcctgcta 6840
   gcccatgctc tgatgttaat gacatggaag gcacccctcc cgaggaaatc gcaactgcac 6900
   aacccctatt acaccccaat tcagcaggaa gcagttagag cattcatcag ccaacctccc 6960
   caacagcact tgggttttcc tattgagagg gggtactgag agacaggact agctggatgt 7020
   cctaggctga ctaagaatcc ctaagcctag ctgggaaggt gaccacatcc acctttaaat 7080
   acggggcttg caacctagct cacacccaac agatcagaga gctcgttaaa atgctaatta 7140
   ggcaaaaaca ggaggtaaag aaatagccaa tcatctattg cctgagagca cagcaggagg 7200
   gacaaggatt gggatataat cccaggcatt cgagctggca acagcaaccc cctttgggtc 7260
   ccctcccttt gtatgggagc tgttttcact ctatttcact ctattaaatc ttgcaactgc 7320
   actcttctgg tgcatgtttg ttactgcttg agctgaactt tcactcgcca tctaccactg 7380
   ctgttttgcc gccgtcgcag acccactgct gacttccatt cttctggatc cagcagggtg 7440
   tccactgtgc tcctgatcca gtgaggcacc cattgccgct cccgatctgg ctaaaggctt 7500
   gccattgttc ctgcatcgct aagtgcctgg gttcgtccta atcaagctga acactagtca 7560
   ctgggttcca cagttctctt ccatgaccca cgacttctaa tagagctata acactcacct 7620
   tatggcccaa gattccattc cttggaatcc atgaggccaa aaaccccagg tcagagaaca 7680
   tgagacttgc caccatgttg aagtggcctg ctgccatttt ggaagtggcc caccaccatc 7740
   ttgggagctc tgggagcaag gacccctggt aaca 7774
<210> 9
   <211> 7487
   <212> DNA
   <213> Homo sapiens
<400> 9
   tgagaaacag gactagttag atttcctagg ccaactaaga atccctaagc ctagctggga 60
   aggtgatcgc atccaccttt aaacacgggc ttgcaactta gctcacacct gaccaatcag 120
   gtagtaaaga gagctcacta aaatgctaat taggcaaaaa caggaggtaa agaaatagcc 180
   aatcatctat tgcctgacac cacacgggga gggacaatga ttgggatata aacccaggaa 240
   ttcgagctgg caacggcaac tccctttggg tctcctctca ttgtatggga gctctgtttt 300
   cactctatta aatcttgcaa ctgcacactc ttctggtctg tgtttgttat ggcttgagct 360
   gagcttttgc tggctgtcca ccactgctgt ttgctgccgt cgcagacccc ttgctgactc 420
   ccacccctgc ggatctggca gggtgtctgc tgcgctcctg atccagccag gcacccactg 480
   ctgctcccaa tcaggctaaa ggcttgccat tgttcctgca tggctaagtg cccgggttcg 540
   tgctaattga gctgaacact agtcgctggg ttccacagtt ctcttccgtg acccacagct 600
   tctaatagag ctataacact cactgcatgg cccaacattc cattccttgg aatctgtgag 660
   gccaagaacc cccggtcaga gaacaagaag cttgccacca tcttggaagc agcccgccac 720
   cattttggga gctctaagaa caaggacccc ccagtaacat tttggtgacc acgaagggac 780
   ctccaaagca gtgagtaata ttgaaccact tccgcttgct attctgtcct aaccttcctt 840
   agaattggag gaaaataccg ggcacctgtc ggccagttaa gaacgattag cgtggccgcc 900
   agacttaaga ctctggtgtg aggctgtctg ggaaagggct ttctaacaac ccccaaccct 960
   tccgggttgg gagctttggt ctgcctggaa ccagcttcca ctttcaattt tcctggggaa 1020
   tccaagggct gactagaggc agaaagctgt catcccgaac tcctggcatt agacagttga 1080
   gatcgtggcg cagccagaag tctctactca acagtcaccc atgcgtgcac ccctaccttt 1140
   ccttctaacc catacctccc gggtcccaac catgactttc ttgaaagtgt agcccctaaa 1200
   ttctctttac ctctaaatct acttcttctc atccctgctt cctaggtact aatggttcag 1260
   actttcattt cctctagcaa gttctatctc cagagggatc taaggaaggg atctatgctg 1320
   tgtccttagg cccctaggct atgaacccag agagtcttct ccctgttatc tctccccatt 1380
   taggcataca gctctcaaca tggacagtta tgtgggaccc attccctacc acccttgcca 1440
   gggccccaag ttttcaaagg gctagaagaa aaaagagaga aagagagaga gaggcagagg 1500
   ggagagaaag agagagagac aaagagggag tcaaagagag atagaaagag aaagatagaa 1560
   ctagtaaaga aaaaaagtat gccccattcc tttaaaagcc agggtaaatt taaaacctat 1620
   aattgataat tgaaggtctt ctccatgacc ctataacact ccaataccac cttgttttca 1680
   gtgtaaacaa gggtgtagcc cgaaaacact gagaccactg acaacccata gccttcctat 1740
   caaaaatcct taacccagga acccatggat ggcccaaatg cattcaatct gtagcagcaa 1800
   ctgctttgct aacagaagaa agtagaaaag taacttttag agaaaacctc attgtgagca 1860
   cacctcacca gttcagaatt attctaagtc aaaaaagcaa aaaggtagct tactaactca 1920
   aaaatcttaa agtatggggt tattctgtta gaaaaaggtg atttaacatt aaccactgaa 1980
   aattccctta acccagcagg tttcctaatg ggatttaaat cttcattacc atacaaaggt 2040
   ccgaccagac ccagcaggaa ctccctttag gacaggatga tagatggttc ctcctgggtg 2100
   attgaggggg tgaaaaacca caatgggtgt tcagtaattg atagggagac tcttgtggaa 2160
   ggagagttag gaaaattgcc taataattgg tctgctcaaa tgtgcgagct gtttgcactc 2220
   agccaagcct taaagtactt acagaatcaa aaagactcta tctcaatcct gactcaaaat 2280
   gttacctaca ccatctctga catgaatttg cataagaact gttgtttatg ggaatgcatc 2340
   ttgatggggc agctgggttg ttatgaaata ctcaggaacc cagcccaggt ctagaattca 2400
   cctctgagcg caaaggcaat gttggccatg ctggtaaagg accactagaa tccaggagcc 2460
   tggacccctt tctttgtggt caagaaaggc gggaaaacag gtgcaggact gctacatcag 2520
   agagcataac aaatccgata agcagagttc catgagtggt taagcaccct ggaaaggaac 2580
   tcacctctga gtgcaaaggc aatgttaggc acaccagtaa aggaccacta gaatccagca 2640
   gcccagaccc ctttctttgt gatcaagaaa ggcgggaaaa ggggtgcagg actgctacat 2700
   cagtgagcgt aactaatctg ataagcagaa gtccatgggt ggttacgcac cctggaaagg 2760
   aataagcatt aggaccacag aggacactct aagactaatg ctcattggaa aatgactagg 2820
   ggtgctggca tccctatgtt tttttttcag atgggaaaca ttccccccaa ggcaaaaacg 2880
   cccataagat atattctgga gaattcggcc cagagtgtat gtatcttttt tccctgtcag 2940
   acttgaagca aacctaggta aattatcaga tagccctgat ggctatattg atgctttaca 3000
   agggttagga caatcctttg atctaacatg gagagatata ctgttactgc tagatcagac 3060
   actaatccca aatgaaagaa gtgccaccat aactgcagcc agagagtttg atgatctctg 3120
   gtatctcagt caggtcaatg ataggatgac aacagaagaa agaaaacaat tccccacagg 3180
   ccagcaggca gttcccagcg tagaccttca ttgggacaca gaatcagaac atggagattg 3240
   gtgccgcaga catttactaa cttgcgcgct agaagcacta aggaaaacta ggaagaagcc 3300
   tatgaattat tcaatgatgt ccactataac acagggaaag gaagaaaatc ctactgcctt 3360
   tctggagaga ctaagggagg cattgagaaa gcatacctct ctgtcacctg actctattga 3420
   aggccaacta atcttaaagg ataagttttc cactcagtca gctgcagaca ttagaaaaaa 3480
   acttcaaaag tctgcgttag gccgggagca aaacttagaa accctattga acttggcaac 3540
   ctcagttttt tatgatagag atcaggagga tcaggtggaa tggacaaatg agattttaaa 3600
   aaaaggccac cactttagtc atggccctca ggcaagcaga ctttggacac tctggaaaag 3660
   ggaaaagctg ggcaaatcga atgcctaata agacttgctt ccagtgtggt ctacaaggac 3720
   actttaaaaa agattgtcca aatagaaata agccaccccc tcgtccatgc tccttatgtc 3780
   aagggaatca ctggaaggcc tactgcccca ggggatgaag gtcctctgag tcagaagcca 3840
   ctaaccagat gattcagccc caggactcag ggtgcccagg gcaagcgcca gcctatgcca 3900
   tcaccctcac agagccctgg gtatgcttga ccattgaggg tcaggaggtt aactatctcc 3960
   tggacactgg cgtggccttc tcagtcttac tctcctgtcc cggacaactg tcctccagat 4020
   ctgtcactat ccgagggttt ctacgacagc cagccactag atacttctcc cagccactaa 4080
   gttgtgactg ggaactctaa ctcttttcac atgtttttct aattatgcct gaaagcccca 4140
   ctcctttgtt agggaaagac attctagcaa aagcaggggc cattatacac ctgaacatag 4200
   gagaaggaac acctgtttgt tgtcccctgc ttgaagaagg aattaatcct gaagtctgga 4260
   caacagaagg acaatacaga tgagcaacaa atgcctgtcc tgttcaagtt aaactaaagg 4320
   attatgcctc ctttccctac caaaggcagt acccccttag acccgaggcc caacaaggac 4380
   tccaaaagat tgttaaggac ctaaaagctc aaagcctagc aaaaccatgc agtagcccct 4440
   gcaatactcc aattttagga gtacagaaaa ccaacagaca gtggaggtta gtgcaagatc 4500
   tcaggattat caatgaggct gttgttccta acccttatac tctgctttcc caaataccag 4560
   aagaagcaga gtggtttaca gtcctggacc ttaaggatgg ctttttctgc atccctgtac 4620
   atcctgactc tcaattcttg tttgcctttg gagatccttc gaacccaatg tctcaactca 4680
   gcttgactgt tttaccccaa gggttcaggg atagccccca tctagttggc caagcattag 4740
   ccgagccagt tctcctacct ggacactctt gtcctctggt acatggatga tttattttta 4800
   gctgcccgtt cagaaacctt gtgccatcaa gccacccaag tgctcttaaa tttcctcgcc 4860
   acctgtggct acaaggtttc caaaccaaag gctcagctct gctcacagca ggttaaatac 4920
   ttagggctaa aattatccaa aggcaccagg gccctcagtg aggaatgtat ccagcctgta 4980
   ttggcttatc ctcatcccaa aaccctaaag caactaagag ggttccttgg cataacaggt 5040
   ttctgccaaa tgtggattcc caggtacggt gaaatagcca ggccattata taccctaatt 5100
   aaggaaactc agaaagccaa cacccattta ttaagatgga cacctgaagc agaagcagct 5160
   ttccaggccc taaagaaggc cctaacccaa gccccagtgt taagcttgcc aacggggaag 5220
   acttttcttt atatgtcaca gaaaaaacag gaatagctct aggagtcctt agacaggtcc 5280
   aagggatgag cttgcaacct gtggcatacc tgagtaagga aattgatgta gttgcaaagg 5340
   gttgacctca ttgtttacag gtagtggcgg cagtagcagt cttagtatct gaagcagtta 5400
   aaataataca gggaagagat cttactgtgt ggacatctca tgatgtaaac ggcgtactca 5460
   cttctaaagg agacttgtgg ctgtcagaca accgtttact taaatatcag gctctattac 5520
   ttgaagggcc agtgctgcga ctgcccactt gttcaactct taacccagcc acatttcttt 5580
   cagacaatga agaaaagata gaacataact gtcaacaggt gattgctcaa acctacggcg 5640
   ctcgagggga ccttctagag gttcccttga ctgatcccaa cctcaacttg tatactgatg 5700
   gaagctcctt tgtagaaaaa ggactttgaa aggtggggta tgcagtggtc agtgataatg 5760
   gaatacttga aagtaattcc ttcactccag gaactagtgc tcagctggca gaactaatag 5820
   ccctcactca ggcactagaa ttaggagaag gaaaaagggt aaatatatat gcagactcta 5880
   agtatgctta cccagtcctc cacgcccaca cagcaatatg gagagatagg aaattcctaa 5940
   cttctgaggg aacaccgatc aaacatcagg aagccattag gagattatta ttggctgtac 6000
   agaaacctaa agaggtggca gtcttacact gctggggtca tcagaaagga aaggaaaagg 6060
   aaatagaaag gaaccaccaa gtggatattg aagccaaaag agccacaagg caggccctcc 6120
   attagaaatg cttatagaag gatccctagt atggggtaat cccctccggg aaaccaagcc 6180
   ccagtactca gcaggagaaa tagacacgag gacatagttt cctcccctca ggatggctag 6240
   ccaccgaaaa agggaaaata cttttgcctg cagctaatca atggaaatta cttaaaaccc 6300
   ttcaccaaac ctttcacttg ggcatggata gcatctatca gatggccaat ttattattta 6360
   ctggaccagg ccttttcaaa actatcaagc agatagtcag ggcctgtgaa atgtgccaaa 6420
   gaaataatcc cctgcacttc aagccataca tttcaatccc tgtatcttta acctcctgtt 6480
   gtttgtctct tccagactca aagctgtaaa actgcaaatg gttcctcata tggagcccca 6540
   gatgcagtcc atgactaaga tctaccacag agccctagac cggcctgtta gcccatgctc 6600
   cgatgttgat gacatcaaag gcacaccttc cgaggaaatc tcaactgcac gacccctact 6660
   aagccccaat tcagcaggaa gcagttaaga gcagtcgttg gctaacatcc ccaatagtat 6720
   gtgggttttc ctgttgagag gggggactga gagacaggac tagctggatt tcctaggcca 6780
   actaagaatc cctaagccta gttgggaagg tgaccgcatc cacctttaaa cacggggctt 6840
   gcaacttagc tcacacccga ccaatcaggt agtaaagaga gctcactaaa atgctaatta 6900
   ggcaaaaaca agaggtaaag aaatagccaa tcatctatcg cctgagagca cagtggggag 6960
   ggacaatgat cgggatataa acccaggcat tcgggccggc aacggcaacc cccattgcgt 7020
   cccctcccat tgtatgggag ctctgttttc attctattaa atcttgcaac tgcacactct 7080
   tctggtctat gtttgttatg gctcgagctg agctttcgct cgctgtccac cactgctgtt 7140
   tgccgccatc gcagacccac cactgacttc cacctctgca gatctggcag ggtgtccgct 7200
   gtgctcctga cccagcgagc cacccattgc tgctcccaat caggctaaag gcttgccatt 7260
   gttcctgcat ggctaagagc ccagggttcg tcctaatcga gctgaacgct agtagctggg 7320
   ttccacagtt ctcttccgtg acccacggct cctaatagag ctataacact caccacatgg 7380
   cccaaggttc cattcattgg aatccgtgag gccaagaacc cccggtcaga gaacaagaag 7440
   cttgccacca tcttggaagc tctaaaaaca gagacacccc agtaaca 7487
<210> 10
   <211> 5470
   <212> DNA
   <213> Homo sapiens
<400> 10
   tgagagacag ctggatttcc taggccgact aagaatccct aagcctagct gggaaggtga 60
   ccgcatccac ctttaaacac agggcttgca acttagctca cacccaacca atcagagagc 120
   tcactaaaat gctaattagg caaaaacagg aggtaaagaa atagcaagtc atctattgcc 180
   tgagagcaca gtgggaggga caaggaccag gatataaacc caggcatttg agccagcaac 240
   ggcaacctcc tttgagtccc ctccctttgt ataggagctc tgttttcact gtgtttcact 300
   ctattaaatc ttgcaattgc actcttctgg tccatatttg tcacggcttg agctgagctt 360
   tcacttgccg tccaccacta ctgtttgctg ctgtcacaga cccgccgctg actcccatcc 420
   cgctgctgac tcccatccct ccggatccgg cagggtgtcc gctgtgctcc tgatccagca 480
   agactcccat tgccactccc gatagtgcta aaggcttgcc attgttcctg catggctaag 540
   tgcctgggtt cgtcctaatc cagctgaaca ctagtcactg ggttccacgg ttctcttcca 600
   tgacccgcgg cttctaatag agctataaca ctcaccacat ggcccaatat tccattcctt 660
   ggaatccgtg aggccaagaa ccccaggtca gagaacacga ggcttgccac catcttggaa 720
   gcagcctgcc accatcttgg aagtggctca ccaccgtctt gggagttctg tgaacaagga 780
   cccctggtaa cattttggcg accacgaagg gacatccaaa gctgtgagta atattggacc 840
   actttcgctt gctattctgt tctatcctta gaactggagg aaaatactgg gcacctgtcg 900
   ccagttaaaa atgattagca tggccgccgg acttaagact caggtgtgag gctatctggg 960
   aaagggcttt ctaacaaccc ccaagccttc tgttgggaac tttggtctgc ctggagccag 1020
   cttccacttt caattttctt ggggaagcca agggctgact ggaggcagaa agctgttgtc 1080
   ccgaactccc ggcagtagcc ggttgagatc atggcgcagc cagaagtctc tactcggcag 1140
   tcgcccatgc gtgcgccctt acctttcctt ctgaattata cctccggggt cccgactccg 1200
   actttcttga gagtttagcc ccaaaattct ccttacctct gaatctactt cctttgatcc 1260
   ctgcctcctg cctcctaggt actaatagtt cagactttca tttcctctag caagttgtgt 1320
   ctccaaaggg atctaaggag gctctatgct gtgtccttag gcacctaggc tataacccag 1380
   ggagtcttat ccctggtatc cctcccgatt taggtataca gctcttgaca tgggcagtta 1440
   tgtgggacct gttccccacc acccttgtga gggccccaag tttgtaatgg ctaagaaaga 1500
   gagacggaga gagagagaga cggagaaaga gacaaagagg gagtcaaaga gaaaaagaaa 1560
   gaaaaagata gaaatagtta aaaaaaaaaa aaagtgtgcc ctattccttt aaaagccagg 1620
   gtaaatttaa aacctgtaat tgataattgc cactttgttg tcagtgtaaa taagggcgta 1680
   gcaaatcctt aacccagtaa cccgcggata ggccaaatgc attcagtcgg tagcggcaac 1740
   agctttgcta aaagtagaaa agtaactttt agaggaaacc tcattgtgag cacacctcac 1800
   cagttcagag ttattctaag taaaaaaaaa aaaaaaaaaa aaagcaaaaa ggtagcttac 1860
   taactcaata atcttaaagt atggggctac tatgctagaa aagggtaatg taactccaac 1920
   cactgataac tcccttaacc cagcagattt cctaacaggg gatttaaatc ttaattacca 1980
   cacgaaggtc cgaccagacc taggaggaac tcccttcagc acaggacgat agatggttcc 2040
   tcccaggtga ctgaggaaaa aactacaatg ggtattcagt aattggtatg gagactcttg 2100
   tggaagcaga gttaaaaatt tgcctaataa ttggtctcct caaatgtgcg agctgtttgc 2160
   actcagccaa gccttaaagt acttacagaa tcaaaagact atctcaatcc tgactcaaaa 2220
   ggttagctac acagtctctg aaatgaattt gcagaagaac tgttgtttat gggaatgcat 2280
   cttgatgggg cagctgggtt gttatgaaat actcaggaac ccagcccagc tctaggactc 2340
   accgctgagc gcaaaggcaa tgttgggcac gctggtaaag gaccactaga atccagcagc 2400
   ccaggcccct ttctttgtgg tcaagaaagg caggaaaagg agtgcagaac tgctacattg 2460
   gtgagcgtaa ctaatccaat aagcagaggt ccatgagtgg ttatgcacgc tggaaaagaa 2520
   taagcattag gcccttagag gatgctctag gactaatgct catcggaaaa tgactagggg 2580
   tgctggcatc cttatgttct ttcttcagat gggaaacgtt ccccccaagg caaaagcgcc 2640
   cctaagatgt attctggaga attagaacca atttgaccct cagatgtcaa gaaagaaacg 2700
   acttatattc ttctgcagta ctgcctggcc acgatatcct cttcaagggg gagaaacctg 2760
   gcctcctgag ggaagtacaa attataacac catcttacag ctagacctct tttgtagaaa 2820
   agaaggcaaa tggagtgaag tgccatatgt gcaaactttc ttttcattaa gagacaactc 2880
   acaattatgt aaaaagtgtg gtttatgtct tacaggaagc cctcagagtc tacctcccta 2940
   tcccagcatt cccccgactc cttccccaac taataagcac accccttgaa cccaaacagt 3000
   ccaaaaggag atagacaaac aggtaaacaa tgaaccaaag agtgtcagta ttccccgatt 3060
   atgccccttc caagcagtgg gaggaggaga attcggccca gccagagtgc atgtaccttt 3120
   ttctctctca gacttaacgc aaattaaaat agacttaggt aaattctcag ataaccctga 3180
   tggctacatt gatgttttac aagggttagg gcaatccttt gatctgacat ggagagatat 3240
   aatgttactg ctaaatcaga cactaacccc aaatgagaga agtgccgccg taactgcagc 3300
   ccgagagttt ggtgatctct ggtatctcag tcaggtcaat gataggatga caacagagaa 3360
   aagagaacga ttccccacag gccagcaggc agtttccagt gtagaccctc attaggacac 3420
   agaatcagaa catggagatt ggtgccacag atatttgcta acttgagtgc tagaaggact 3480
   aaggaaaact aggaagaagc ctatgaatta ttcagtgatg tccactataa cacaaggaaa 3540
   ggaagaaaat cctactgcct ttctggagag agtaagggag gcattaagga agcatacctc 3600
   cctgtcacct gactctattg aaggccaact aatcttaaag gataagtttg tcactcagtt 3660
   agctgcagac attagaaaaa aacttcaaaa gtccgactta ggcctggagt acggctgagt 3720
   gcccaatttg gcagcaggca agaccaacac tgagcccttc atatggcacc atgctttgtg 3780
   gtgatcagcc aactacttga tggcaggttg attatattgg acatctttca tcagagaaat 3840
   ggcagtggtt tgtccttcct ggaatagaca cttattctcg atatgggttt gtctatcctg 3900
   caggcaatgc ttctgccagg agtaccatct gtggactcat ggaaagcctt atccaccatc 3960
   atggcattcc acacagcatt gcctctaaac aaggcactta ttttatagct aaggaagtgt 4020
   ggcagtgggc tcatgctcat ggaattcact gattgtatct tgttgcccat tatcttaaag 4080
   cagctggatt gatagaacag tggaaaggcc atttgaaatc acaattacac caccaactag 4140
   gtgacaatac tttgcagggc tcggcaaagt tctcttgaag gctgagtatg tcctgaatca 4200
   gcatccaata tatggtactg tttccctcat agccagcatt cacaggccta agaatcaagg 4260
   ggtagaagta gaagtggcac cactcaccat cactcctagt gacccactag caaaaatttt 4320
   acttccagtt cccccaacat tatgttctgc tggccttagt tccagaggga agaattctgc 4380
   caccagtcga cacaagaatg ataccattaa actgaaagtt aaaattgcca cctggccact 4440
   ttgggctcct cccacctcta agtcaacagg tcaagaaagg agttacagtg ttgacttggg 4500
   tgattgacct ggactatcaa gatgaaatca ggttactact ccacagtgga ggtaaggaag 4560
   aatatgtgtg gaatacagga gatcccttag gccgtctttt agtactacca tgccctgtga 4620
   ttaaggtcag tggaaaacta caacaatcca atctaggcag gactacaaat ggcccagact 4680
   cttcaggaat gaagggttgg gtgacttcac caggtaaaaa aataacagcc tgctgaggtg 4740
   ctagctgaag gcaaagggaa tacagaatgg ttagtagaaa aaggtagtca tcaataccag 4800
   ctatgaccac aagaccagtt gcagaaatga gacctgtaat tgtcatgtgg atttcctcct 4860
   tacatgtttg tgcatgtata cacttctact aagaaaatac ctttatttat ttcctttgct 4920
   tttcccttat caagtgacat tattaacttc atatcagcag ttaagtgtta ttaactttat 4980
   gtaatagcat ttcggttaat aattcacttc tggttgtatg aaggatagcc gtattaagtt 5040
   aggtgtaatt atgacatcat tattgtcttt atttgaagat tatgtgtaat ttcaggagat 5100
   gtgtatgggt tcaagttgac aagggatgga cttgtgatgg ctaatgttga gtgtcaactt 5160
   gactgaggat gcaaagtatt gttcctgggt gtgtctgtga gggtgttgcc aaaggagatt 5220
   aacatttgtg tcagtgaact gggagatgca gacccacccg caatctgggt gagcaccatg 5280
   taatcagctg ccagagcagc tagaataaag caagcagaag aaggtggaag gagctgactt 5340
   gctgagtctt ctagtattct tcgttcttct atgctggttg cttcctgccc ccaaacatca 5400
   gtctgcaagt tcttctgctt ttggactctt ggacttacac cagtggtttg ccagggactc 5460
   tcgggccttc 5470
<210> 11
   <211> 783
   <212> DNA
   <213> Homo sapiens
<400> 11
   tgagagacag gactaactgg atttcctagg ccgactaaga atccctaagc ctagctggga 60
   aggtgaccgc atccatcttt aaacacgggg cttgaaactt agctcacacc taaccagtca 120
   gagagctcac taaaatgcta attaggcaaa aaacaggagg taaagaaata gccaatcatc 180
   tattgcctga gagcacagcg ggagggacaa ggatcgggat ataaacccag gcattcgagc 240
   cagcaatggc aacccccttt gggtcccctt cccttgtatg ggagctctgt tttcactcta 300
   tttcactcta ttaaatcttg caactgcact cttctggtcc atgtttgtta cggctcgagc 360
   tgagctttgg ctcgccatcc accactgctg tttgccgccg tcgcacacct gctgctgact 420
   cccatccctc cggatccagc agggtgtgtc cgctgtgctc ctgatccagc gaggtgccca 480
   ttgccgctcc tgattggact aaaggcttgc cattgttcct gcacggctaa gtgcccgggt 540
   tcgtcctaat ccagctgaac actagtcact gggttccacg gttctcttcc ttgacccacg 600
   gcttctaata gagctataac actcaccgca tggcccaaga ttccattcct tggaatctgt 660
   gaggccaaga accccaggtc agagaacacg aggcttgcca ccatcttgga agcggcctgc 720
   caacatcttg gaagtggctc gccaccatct tgggagctct gtgagcaagg acccctggta 780
   aca 783
<210> 12
   <211> 7542
   <212> DNA
   <213> Homo sapiens
<400> 12
   tgagagacag gactagctgg atttcctagg ccgactaaga atccctaagc ctagctggga     60
   aggtgaccgc ttccaccttt aaacacgggg cttgcaactt agctcacacc cgaccaatca    120
   gatagtaaag agagcacact aaaatgctaa ttaggcaaaa acaggaggta aagaaatagc    180
   caatcatcta ttgcctgaga gcaaagcggg agggacaatg atcgggatag aaacccaggc    240
   attcaagccg gaatggctac cctctttggg tcccctccct ttgtatggga gctctgtttt    300
   cactctattc aatcttgcaa ctgcactctt ctggtccgtg tttgttacag cttgagctga    360
   gctttcgctc gccttccacc actgctgttt gccgccatcg cagacctgcc gtgctgactt    420
   ccatccctct agatctggca gggtgtccgc tgtgctcttg atccagcgag gcgcccattg    480
   ccgctcccga ttgggctaaa ggcttgcaat tgttcctgca cgctaagtgc ctgggttcat    540
   cctcatcaag ctgggttcca cggttctctt catgacccgc agcttctaac agagctataa 600
   aactctgtgc atggcccaag attccattcc ttggaatctg tgaggccaag aaccccaggt 660
   cagagaacag gaggcttgcc accatcttgg aagtggctcg ccaccatctt aggagctctg 720
   tgagcggaga cccccacccc ccggtaacat tttggcgacc acgaagggac ctccaaagcg 780
   gtgagtaata ttggatcact ttcgcttgct attctgtcct atccttcttt agaattggag 840
   gaaaatactg ggcacctgtc ggccagttaa aaacaattag cgtggctgcc cgacttaaga 900
   ctcaggtgtg aggctatctg gggaagggct ttctaacaac ccccaaccct tctgggttgg 960
   ggacgttggt ctgccccttc cactttcaat tttcttgggg aagccaaggg tcgactagag 1020
   gcagaaagct gtcgtccgga actcctggca gtagccggtt gagatcatgg cgcagccaga 1080
   agtctctact caacagtcgc ccatgcgtgc gctcctacct ttcctcctga cccatacctc 1140
   ctgggtcccg acgatgactt tcttgaaagt gtagccccaa aattctgctt acctctgaat 1200
   ctacttcccc tgatccctgg ctcctaggta ctaatggttc agtttcattt cctctagcaa 1260
   gttgtatctc caaagggatc taaggaagct ctacgctgcg tccttaggca tctaggctat 1320
   aaacccagga agtcttgtcc ctggtgtccc tcccgattta ggcatacagc tctcgacatg 1380
   ggcagttatg tgggacccgt tccccatcac ccttgtcaag gccccaagtt tgtaatggct 1440
   aagaggagag agagagaaag agagagagac ggaggggaga gagagagaga gagatggagg 1500
   ggagagagag agagagagac ggaggggaga gagagagaga gagagagacg gaggggagag 1560
   agagagagac ggaggggaga gagagagaga tggaggagag aaagacaaag ggagtcaaag 1620
   agaaaaagaa agagaaagac agaaatggta aaacaaacaa aaaacagcgt gccctattcc 1680
   tttaaaagcc ggggtaaatt taaaacctat aattgataat tgaaggtctt ctccatgacc 1740
   ctataatact ccaatactac cttgttgtca gtgtaaacaa gggcgtagcc tgaaaacact 1800
   gagaccactg acaacctgca gctttcctat caaaaaatcc ttaacccagt aaccggcaga 1860
   tgcattcaat ctgtagcagc aactgttttg ctaacagaag aaagtagaaa agtaactttt 1920
   agaggaaacc tcattgtgag cacaccttac cagttcagaa ttattctaag tcaaaaaagc 1980
   aaaaaggtag cttactaact caaaaatctt aaagtatggg gctattgtgt ttaaaaaaaa 2040
   aaaaaggtaa tttaacacca accactgata attctcttaa cccagcaggt ttcctaacag 2100
   gggatttaaa tcttaattac catacaaagg tctgaccaca cctaggagga actcccttca 2160
   ggacaggact atagagggtt cctcccaggt gattgaggaa aaaaccacag tgggtattca 2220
   gtaattgata gggagactct tgtggaagca gagttagaaa aattgcctaa taaatggtgt 2280
   cctcaaaagt gtgagctgtt tgcactcagc caagccttaa agtacttaca gaatcgtaaa 2340
   aactatctca atcctgactc aaaagtttac ttacaccctc tctgaaatga atttacataa 2400
   gaactgcttt tttgggaatg catcttgatg gggcagctgg gtggttatga aatactcagg 2460
   aaaccagccc agctctagga cacatccctg agcacaaagg caatgttggg cacgctggta 2520
   aaggaccact agaatccagc agcctggact cctttctttg tggtcaagaa aggcaggaaa 2580
   acaggtgcag gactgctaca tcagtgagca taactaatct gataagcaga gggccttggg 2640
   tggttacaca ccctggaaag gaattcaact ctgagcgcaa aggcaatgtt gggcacattg 2700
   gtaaaggacc actagaatcc agcagcccag gcccctttct ttatggtcaa gaaaggcggg 2760
   aaaaggggtg caggactgtt acctcggtga gcgtaactaa tccgataagc agaggtccat 2820
   gggtgattac gcaccctgaa aagaataagc attaggccct taaaggatgc tctaggacta 2880
   atgctcattg gaaaatgact aggggtgctg gcatccctat gttcttttct cagacgggaa 2940
   atgttctcca ccctccccaa ggcaaaaaca cccctaagat gtattctgga gaattgggac 3000
   caatttgacc cccagacgct aagaaagaga tgacttatgt tcttctgcag taccacctgg 3060
   ccacgatatc ctcttcaagg gggagaaacc tggcctcctg agggaagtat aaattataac 3120
   accatcttac agctagacct cttctgtaga aaggagggca aatggagtga agtgccatat 3180
   gtgcaaactt tcttttcatt aagagacaac ttgcaattat gtaagaagtg tgatttatgc 3240
   cctacaggaa gccctcagag tctacctccc taccccagca tccccctgac tccttctcca 3300
   actaataagg aacccccttc aacccaaacg gtccaaaagg agatagacaa aggggtaaac 3360
   aatgaaccaa agcgtgccaa tgttccctga ttatgccccc tctaagcagt gggaggagga 3420
   gaatttggcc cagccagtgt gcatgtgcct ttttctctct cagacttaaa gcaaattaaa 3480
   atagacctag gtaaattctc agataaccct gatggctata ttgatgtttt ataagggtta 3540
   ggataatcct ttgatctgac atggagagat ataatgttac tgctagatca gacactaacc 3600
   ccaaatgaga caagtgccgc cataactgca gcctgagagt ttggcgatct ctggtatctc 3660
   actcgggtca atgataggag gacaacagag gaaagagaat gattccccac agaccagcag 3720
   gcagttccca gtgtagaccc tcactgggac acagaatcag aacatggaca ttggtgctgc 3780
   agacatttgc taacttacat gctagaagga ctaaggaaaa ctaggaagaa gcctacgaat 3840
   tattcaatga tgtccactat aacacaggga aaggaagaaa atcctactgc ctttctggag 3900
   cgactaaggg aggcattgag gaagcatact tccctgtcac ctgactctat tgaaggccaa 3960
   ctaatcttaa aggataagtt tatcactcag tcagctgaag acattaggaa aaaacttcaa 4020
   aagtctgcct taggcccaga gcaaaactta gaaaccccat tgaacttggc aacctcggtt 4080
   ttttataata gagatcagga ggagcaggcg gaacaggaca aacggggtaa aaaaaaggcc 4140
   accgctttag ttatggccct caggcaagtg gactttggag gctctggaaa agggaaaagc 4200
   tgggcaaatc gaatgcctac tagggcttgc ttccagagtg gtctacaagg acactttgaa 4260
   aaagattgtc caagtagaaa taagtcgccc cttcgtccat gccccttata tcaagggaat 4320
   cactggaagg cccactatcc caggggacaa atgtcctctg agtcagaagc cactaaccag 4380
   atgatccagc agcaggactg agggtgccca gggcaagcac tagcccatgc cgtcaccctc 4440
   acagagcccc aggtatgctt gaccattgag ggccaggagg ttaactgtct cctggacact 4500
   agcacggcct tctcagtctt actctccttt cccggacaac tgtcctccag atctgtcact 4560
   atccgagggt tcctaggaca gtcagtcact agatacttat cccagtcact aagttgtgac 4620
   tggtgaactt tactcttttc acatgctttt ctaattatcc ctgaaagcac cactcccttg 4680
   ttagggcgag acattctagc aaaagcaggg gccattatac acctgaacat aggagaagga 4740
   acacctgttt gttgtcccct gcttgaggaa ggaattaatc ccgaagtctg ggcaacagaa 4800
   ggacaatacg gacgagcaaa gaatgcctgt gctgttcaag ttaaactaaa ggattccgcc 4860
   tcctttccct accaaaggca gtaccccctt agacctgagg cccaacaagg actccaaaag 4920
   attgttaagg acctaaaagc ccatggccta gtaaaaccat gcaatagccc ctgcaatact 4980
   ccaattttag gagtacagaa acccaacaga cagtggaggt tagtgcaaga tctcaggatt 5040
   atcattgagg ctgttgttcc tgtatagcca gctgtaccta acccttatac tctgctttcc 5100
   caaataccac aggaagcaga ggggtttaca gtccggggcc ttaaggacac ctttttctgc 5160
   atccctgtat atcctgactc tcaattcttg tttgcctttg aagatccttc aaactcaacg 5220
   tctcaactca cctggaatgt tttaccccaa gggttcaggg atagccccca ttagcccaag 5280
   acttgagcca gttcttatac ctggacactc ttgtcctttg gtacgtggat gatttacttt 5340
   tagccacctg ttcagaaacc ttgtgccatc aagccaccca agcactcttt aatttcctcg 5400
   ccacctgtgg ctacaggttt ccaaaccaaa ggctcagctc tgctcacagc aatttaaatg 5460
   cttagggcta aaattatcca aaggcaccag ggccctcagt gaggaaagta tccggcctat 5520
   actggcttat cctcatccca aaaccctaaa gcaactaaga gtgttccttg gcataacggg 5580
   tttctgccga atatggattc ccaggtacag cgaaatagcc agaccattat atacactaat 5640
   taaggaaact cagaaagcca atacccattt ggtaagatgg acacctgaag cagaagcaga 5700
   tttccaggcc ctaaagaagg ccctgaccca agccccagtg ttaagcttgc caatggggca 5760
   agacttttct ttatatgtca cagaaaaaac aggaatagct ccaggagtcc ttacgcagat 5820
   ccaagggacg agcctgcaac ccatggcata cctgagtaag gaaattagtg gcaaagggtt 5880
   ggcctcattg tttatgggta gtggcagcag tcacagtctt agtaactgaa gcagttaaaa 5940
   tgatacaagg aagagatctt actgtgtgga catctcatga tgtgaatggc atactcactg 6000
   ctaaaggaga cttgtgactg tcagacaact gtttacttaa atatcaggct ctattacttg 6060
   aagggccagt gttgcgactg tgcacttgtg caactcttaa cccagccaca ttgcttccag 6120
   acaatgaaga aaagatagaa cataactgtc aacaaataat tgctcaaacc tacactgctc 6180
   gaggggacct tttagaagtt cccttgactg atcccgatct caacttgtat actgatggaa 6240
   gttcctttgc agaaaaagga cttcaaaagg cggtgtatgc agtagtcctt caaaatcgaa 6300
   gagctttaga attgctaatc actgagagag gggaacgttt tttattttta ggggaagaat 6360
   gctgttatta tgttaatcaa ttcggaatca tcaccaagaa agttaaagaa attcaagatc 6420
   gaatacaacg tagaacagag gagcttaaaa aacactggac cctggggcct cctcagccaa 6480
   tggatgccct ggattctccc cttcttagga cctctagcag ctatatttct actcctcttt 6540
   ggaccctgta tctttaacct ccgtgttaag tttgtctctt ccagaatcga agatgtaaaa 6600
   ctacaaatcg ttcttcaaat ggacccccag atgcagtcca tgactaagat ctactgagga 6660
   cccctggacc agcctgctag cccatgctcc aatgttaatg acattgaagg cacccctccc 6720
   aaggaaatct caactgcaca acccctacta tgctccaatt cagcaggaag cagttacagt 6780
   ggtcctcggc caacctcccc aacagcattt gtattttcct gttgggaggg ggcactgaga 6840
   gacaggacta gctggatttc ctaggctgac tgagaatccc taagcctagc tgggaaggtg 6900
   accacttcca cctttaaaca cagggcttgc aacttagctc acaccctacc aattggatag 6960
   taaagagagg tcactaaaat gctaattagg caaaaacagg aggtaaagaa atagccaatc 7020
   atccattgcc tgagagcaca gcgggaggga caatgaccag gatataaacc caggcattcc 7080
   agcctgcaac ggcaaccccc tttgggtccc ctctctttgt atgggagctc tgttttcact 7140
   ctattcaatc ttgcaactgc actcttctgg tccgtgtttg ttacggctca agctgagctt 7200
   ttgctcacca tccaccactg ctgtttgccg ccgttgcaga cccgtcgctg acttccatcc 7260
   ctccagatct ggcagggtgt ccactgtgct cctgatccag cgaggcaccc attgccactc 7320
   ccgatcaggc taaaggcttg ccattgttcc tgcacagcta agtgcctggg ttcgtcctaa 7380
   tcaagctgaa cactagtcac tgggttccat ggttctcttc catgacccat ggcttctaat 7440
   agagctataa cactcaccgc atggcccaag attccattcc ttggaatccg tgaggccaag 7500
   aaccccaggt cagagaacac gaggctgccg ccatcttgga ag 7542
<210> 13
   <211> 10288
   <212> DNA
   <213> Homo sapiens
<400> 13
   cctggggcgg gcttcctttc tgggatgagg gcaaaacgcc tggagataca gcaattatct      60
   tgcaactgag agacaggact agctggattt cctaggccga ctaagaatcc ctaagcctag     120
   ctgggaaggt gaccacgtcc acctttaaac acggggcttg caacttagct cacacctgac     180
   caatcagaga gctcactaaa atgctaatta ggcaaagaca ggaggtaaag aaatagccaa     240
   tcatctattg cctgagagca cagcaggagg gacaacaatc gggatataaa cccaggcatt     300
   cgagctggca acagcagccc ccctttgggt cccttccctt tgtatgggag ctgttttcat     360
   gctatttcac tctattaaat cttgcaactg cactcttctg gtccatgttt cttacggctc     420
   gagctgagct tttgctcacc gtccaccact gctgtttgcc accaccgcag acctgccgct     480
   gactcccatc cctctggatc ctgcagggtg tccgctgtgc tcctgatcca gcgaggcgcc     540
   cattgccgct cccaattggg ctaaaggctt gccattgttc ctgcacggct aagtgcctgg    600
   gtttgttcta attgagctga acactagtca ctgggttcca tggttctctt ctgtgaccca    660
   cggcttctaa tagaactata acacttacca catggcccaa gattccattc cttggaatcc    720
   gtgaggccaa gaactccagg tcagagaata cgaggcttgc caccatcttg gaagcggcct    780
   gctaccatct tggaagtggt tcaccaccat cttgggagct ctgtgagcaa ggaccccccg     840
   gtaacatttt ggcaaccacg aacggacatc caaagtggtg agtaatattg gaccactttc    900
   acttgctatt ctgtcctatc cttccttaga attggaggaa aataccgggc acttgtcggc    960
   cagttaaaaa cgattagtgt ggccaccgga cttaagactc aggtgtgagg ctatctgggg   1020
   aagggctttc taacaacccc caacccttct gggttgggga cttggtttgc ctcaagccag   1080
   cttccacttt cagttttctt ggggaagccg agggccgact agaggcagaa agctgtcgtc   1140
   ctgaactccc ggcagtagcc ggttgagatc atggtgtagc cagaagtctc aacagtcgcc   1200
   catgcatgca cccctatctt tccttctgac ccatacctcc tgggtcccaa ccacaacttt   1260
   cttcaaagtg tagccccaaa attctcctta cctctgaata tacttcctct gatccctgcc   1320
   tcctaggtac tattggttca gacttccatt tcctctagca agttgtatct ccaaagggat   1380
   ctaaggaagc tctgcgctgc gtccttaggc acctaggcta taacccaggg agtcttatcc   1440
   ctggtgtccc tcccaattta ggcatacagc tcttgacatg ggcagttatg taggacccac   1500
   tccccaccac ccttgccagg gccccaagtt tgtaaatggc tgagggaaaa gagagacaga   1560
   ggagagagag agaaatggag gagaaagaga gagagacaga gaggagagag agacagtgag   1620
   agagacagaa gagagagaga gacaaagagg agagagagag agtcaaagag agaaagaaag   1680
   agaaagaaat agtaaaaaac agtgtgccct attcctttaa aagccagggt aaatttaaaa   1740
   cctgtacttg ataattgaag gtcttctctg tgaccctata gcactccaat ccactttgtg   1800
   gtcagtgtaa ataagagcat aggccgaaag cactgaggcc attgacaacc cgtagcttcc   1860
   ctatcaaaaa tccttaaccc agtaacccgc agatggacca aatgcattca gtcggtagcg   1920
   caactgcttt gctaaaagta gaaaagtaac ttttagagga aacctcattg tgagcacacc   1980
   tcacctgttc agaattattc taataaaaaa agcaaaaagg tagcttacta actcaaaaat   2040
   cttaaagtat ggggctattc tgttagaaaa aggtaatgta actccaacca ctgataattc   2100
   ccttaaccca gcagatttcc taacgggatt taaatcttaa ttaccataca aaggtccgac   2160
   cagacctagg cggaactccc ttcaggacag gacgatagat ggttcctccc aggtgattga   2220
   ggaaaaaaac cacaatgggt attcagtaat tgatacgggg actcttgtgg aagcagagtt   2280
   agaaaaattg cctaataact ggtctcctca aacgtgtgag ctgtttgcac tcagccaagc   2340
   cttaaagtac ttacagaatc aaaagactat ctcaatcctg attcaaaagg ttagctacac   2400
   cctctctgta atgcatttgc ataagaactt gtttatggga atgcatcttg atggggcagc   2460
   tgggttgtta taaaatagga acccagccca gctctaggac tcacccctga gcgcaaaggc   2520
   aatgttgggc atgctggtaa aggaccacta gaatccagca gcccagaccc ctttctttgt 2580
   ggtcaagaaa ggcgggaaaa ggggtgcagg actgctacat cggtaagcat aactaatccg 2640
   ataaacagag gtccatgggt ggttacgcac cctggaaagg aactcacccc tgagcacaaa 2700
   ggcaatgttg ggcacgctgg taaaggacca ctagaatcca gcagcctgga cccctttctt 2760
   tgtggtcaag agaggcagga aaacaggtgc aggactgcaa catcagtgag cataactaat 2820
   tcgataagca gaggtccatg ggtggtgatg caccctggaa agaataagca ttaggaccat 2880
   agaggacact ccaggactaa agctcatcgg aaaatgacta gggttgctgg catccctatg 2940
   ttcttttttc agatgggaaa cgttccccgc aagacaaaaa cgcccctaag acgtattctg 3000
   gagaattggg accaatttga ccctcagaca ctaagaaaga aacgacttat attcttctgc 3060
   agtgccgcct ggcactcctg agggaagtat aaattataac accatcttac agctagacct 3120
   cttttgtaga aaaggcaaat ggagtgaagt gccataagta caaactttct tttcattaag 3180
   agacaactca caattatgta aaaagtgtga tttatgccct acaggaagcc ttcagagtct 3240
   acctccctat cccagcatcc ccgactcctt ccccaactaa taaggacccc ccttcaaccc 3300
   aaatggtcca aaaggagata gacaaaaggg taaacagtga accaaagagt gccaatattc 3360
   cccaattatg acccctccaa gcagtgggag gaagagaatt cggcccagcc agagtgcatg 3420
   tgcctttttc tctcccagac ttaaagcaaa taaaaacaga cttaggtaaa ttctcagata 3480
   accctgatgg ctatattgat gttttacaag ggttaggaca attctttgat ctgacatgga 3540
   gagatataat gtcactgcta aatcagacac taaccccaaa tgagagaagt gccaccataa 3600
   ctgcagcctg agagtttggc gatctctggt atctcagtca ggtcaatgat aggatgacaa 3660
   cagaggaaag agaatgattc cccacaggcc agcaggcagt tcccagtcta gaccctcatt 3720
   gggacacaga atcagaacat ggagattggt gctgcagaca tttgctaact tgtgtgctag 3780
   aaggactaag gaaaactagg aagaagtcta tgaattactc aatgatgtcc accataacac 3840
   agggaaggga agaaaatcct actgcctttc tggagagact aagggaggca ttgaggaagc 3900
   gtgcctctct gtcacctgac tcttctgaag gccaactaat cttaaagcgt aagtttatca 3960
   ctcagtcagc tgcagacatt agaaaaaaac ttcaaaagtc tgccgtaggc ccggagcaaa 4020
   acttagaaac cctattgaac ttggcaacct cggtttttta taatagagat caggaggagc 4080
   aggcggaaca ggacaaacgg gattaaaaaa aaggccaccg ctttagtcat gaccctcagg 4140
   caagtggact ttggaggctc tggaaaaggg aaaagctggg caaattgaat gcctaatagg 4200
   gcttgcttcc agtgcggtct acaaggacac tttaaaaaag attgtccaag tagaagtaag 4260
   ccgccccctc gtccatgccc cttatttcaa gggaatcact ggaaggccca ctgccccagg 4320
   ggacaaaggt cctctgagtc agaagccact aaccagatga tccagcagca ggactgaggg 4380
   tgcctggggc aagcgccatc ccatgccatc accctcacag agccctgggt atgcttgacc 4440
   attgagggcc aggaggttgt ctcctggaca ctggtgcggt cttcttagtc ttactcttct 4500
   gtcccggaca actgtcctcc agatctgtca ctatctgagg gggtcctaag acgggcagtc 4560
   actagatact tctcccagcc actaagttat gactggggag ctttattctt ttcacatgct 4620
   tttctaatta tgcttgaaag ccccactacc ttgttaggga gagacattct agcaaaagca 4680
   ggggccatta tacacctgaa cataggagaa ggaacacccg tttgttgtcc cctgcttgag 4740
   gaaggaatta atcctgaagt ctgggcaaca gaaggacaat atggacgagc aaagaatgcc 4800
   cgtcctgttc aagttaaact aaaggattcc acctcctttc cctaccaaag gcagtacccc 4860
   ctcagaccca aggcccaaca aggactccaa aagattgtta aggacctaaa agcccaaggc 4920
   ctagtaaaac catgcagtaa cccctgcagt actccaattt taggagtaca gaaacccaac 4980
   agacagtgga ggttagtgca agatctcagg attatcaatg aggctgttgt tcctctatag 5040
   ccagctgtac ctagccctta tactctgctt tcccaaatac cagaggaagc agagtggttt 5100
   acagtcctgg accttcagga tgccttcttc tgcatccctg tacatcctga ctctcaattc 5160
   ttgtttgcct ttgaagatac ttcaaaccca acatctcaac tcacctggac tattttaccc 5220
   caagggttca gggatagtcc ccatctattt ggccaggcat tagcccaaga cttgagccaa 5280
   tcctcatacc tggacacttg tccttcggta ggtggatgat ttacttttgg ccgcccattc 5340
   agaaaccttg tgccatcaag ccacccaagc gctcttcaat ttcctcgcta cctgtggcta 5400
   catggtttcc aaaccaaagg ctcaactctg ctcacagcag gttacttagg gctaaaatta 5460
   tccaaaggca ccagggccct cagtgaggaa cacatccagc ctatactggc ttatcctcat 5520
   cccaaaaccc taaagcaact aaggggattc cttggcgtaa taggtttctg ccgaaaatgg 5580
   attcccaggt atggcgaaat agccaggtca ttaaatacac taattaagga aactcagaaa 5640
   gccaataccc atttagtaag atggacaact gaagtagaag tggctttcca ggccctaacc 5700
   caagccccag tgttaagttt gccaacaggg caagactttt cttcatatgt cacagaaaaa 5760
   acaggaatag ctctaggagt ccttacacag atccgaggga tgagcttgca acctgtggca 5820
   tacctgacta aggaaattga tgtagtggca aagggttgac ctcattgttt acgggtagtg 5880
   gtggcagtag cagtcttagt atctgaagca gttaaaataa tacagggaag agatcttact 5940
   gtgtggacat ctcatgatgt gaatggcata ctcactgcta aaggagactt gtggctgtca 6000
   gacaactgtt tacttaaatg tcaggctcta ttacttgaag ggccagtgct gcgactgtgc 6060
   acttgtgcaa ctcttaaccc agccacattt cttccagaca atgaagaaaa gataaaacat 6120
   aactgtcaac aagtaatttc tcaaacctat gccactcgag gggacctttt agaggttcct 6180
   ttgactgatc ccgacctcaa cttgtatact gatggaagtt cctttgtaga aaaaggactt 6240
   cgaaaagtgg ggtatgcagt ggtcagtgat aatggaatac ttgaaagtaa tcccctcact 6300
   ccaggaacta gtgctcagct agcagaacta atagccctca cttgggcact agaattagga 6360
   gaagaaaaaa gggcaaatat atatacagac tctaaatatg cttacctagt cctccatgcc 6420
   catgcagcaa tatggaaaga aagggaattc ctaacttctg agagaacacc tatcaaacat   6480
   caggaagcca ttaggaaatt attattggct gtacagaaac ctaaagaggt ggcagtctta   6540
   cactgccggg gtcatcagaa aggaaaggaa agggaaatag aagagaactg ccaagcagat   6600
   attgaagcca aaagagctgc aaggcaggac cctccattag aaatgcttat aaaacaaccc   6660
   ctagtatagg gtaatcccct ccgggaaacc aagccccagt actcagcagg agaaacagaa   6720
   tggggaacct cacgaggaca gttttctccc ctcgggacgg ctagccactg aagaagggaa   6780
   aatacttttg cctgcaacta tccaatggaa attacttaaa acccttcatc aaacctttca   6840
   cttaggcatc gatagcaccc atcagatggc caaatcatta tttactggac caggcctttt   6900
   caaaactatc aagcagatag tcagggcctg tgaagtgtgc cagagaaata atcccctgcc   6960
   ttatcgccaa gctccttcag gagaacaaag aacaggccat taccctggag aagactggca   7020
   actgatttta cccacaagcc caaacctcag ggatttcagt atctactagt ctgggtagat   7080
   actttcacgg gttgggcaga ggccttcccc tgtaggacag aaaaggccca agaggtaata   7140
   aaggcactag ttcatgaaat aattcccaga ttcggacttc cccgaggctt acagagtgac   7200
   aatagccctg ctttccaggc cacagtaacc cagggagtat cccaggcgtt aggtatacga   7260
   tatcacttac actgcgcctg aaggccacag tcctcaggga aggtcgagaa aatgaatgaa   7320
   acactcaaag gacatctaaa aaagcaaacc caggaaaccc acctcacatg gcctgctctg   7380
   ttgcctatag ccttaaaaag aatctgcaac tttccccaaa aagcaggact tagcccatac   7440
   gaaatgctgt atggaaggcc cttcataacc aatgaccttg tgcttgaccc aagacagcca   7500
   acttagttgc agacatcacc tccttagcca aatatcaaca agttcttaaa acattacaag   7560
   gaacctatcc ctgagaagag ggaaaagaac tattccaccc ttgtgacatg gtattagtca   7620
   agtcccttcc ctctaattcc ccatccctag atacatcctg ggaaggaccc tacccagtca   7680
   ttttatctac cccaactgcg gttaaagtgg ctggagtgga gtcttggata catcacactt   7740
   gagtcaaatc ctggatactg ccaaaggaac ctgaaaatcc aggagacaac gctagctatt   7800
   cctgtgaacc tctagaggat ttgcgcctgc tcttcaaaca acaaccagga ggaaagtaac   7860
   taaaatcata aatccccatg gccctccctt atcatatttt tctctttact gttcttttac   7920
   cctctttcac tctcactgca ccccctccat gccgctgtat gaccagtagc tccccttacc   7980
   aagagtttct atggagaatg cagcgtcccg gaaatattga tgccccatcg tataggagtc   8040
   tttctaaggg aacccccacc ttcactgccc acacccatat gccccgcaac tgctatcact   8100
   ctgccactct ttgcatgcat gcaaatactc attattggac aggaaaaatg attaatccta   8160
   gttgtcctgg aggacttgga gtcactgtct gttggactta cttcacccaa actggtatgt   8220
   ctgatggggg tggagttcaa gatcaggcaa gagaaaaaca tgtaaaagaa gtaatctccc   8280
   aactcacccg ggtacatggc acctctagcc cctacaaagg actagatctc tcaaaactac   8340
   atgaaaccct ccgtacccat actcgcctgg taagcctatt taataccacc ctcactgggc   8400
   tccatgaggt ctcggcccaa aaccctacta actgttggat atgcctcccc ctgaacttca   8460
   ggccatatgt ttcaatccct gtacctgaac aatggaacaa cttcagcaca gaaataaaca   8520
   ccacttccgt tttagtagga cctcttgttt ccaatctgga aataacccat acctcaaacc   8580
   tcacctgtgt aaaatttagc aatactacat acacaaccaa ctcccaatgc atcaggtggg   8640
   taactcctcc cacacaaata gtctgcctac cctcaggaat attttttgtc tgtggtacct   8700
   cagcctatcg ttgtttgaat ggctcttcag aatctatgtg cttcctctca ttcttagtgc   8760
   cccctatgac catctacact gaacaagatt tatacagtta tgtcatatct aagccccgca   8820
   acaaaagagt acccattctt ccttttgtta taggagcagg agtgctaggt gcactaggta   8880
   ctggcattgg cggtatcaca acctctactc agttctacta caaactatct caagaactaa   8940
   atggggacat ggaacgggtc gccgactccc tggtcacctt gcaagatcaa cttaactccc   9000
   tagcagcagt agtccttcaa aatcgaagag ctttagactt gctaaccgct gaaagagggg   9060
   gaacctgttt atttttaggg gaagaatgct gttattatgt taatcaatcc ggaatcgtca   9120
   ctgagaaagt taaagaaatt cgagatcgaa tacaacgtag agcagaggag cttcgaaaca   9180
   ctggaccctg gggcctcctc agccaatgga tgccctggat tctccccttc ttaggacctc   9240
   tagcagctat aatattgcta ctcctctttg gaccctgtat ctttaacctc cttgttaact   9300
   ttgtctcttc cagaatcgaa gctgtaaaac tacaaatgga gcccaagatg cagtccaaga   9360
   ctaagatcta ccgcagaccc ctggaccggc ctgctagccc acgatctgat gttaatgaca   9420
   tcaaaggcac ccctcctgag gaaatctcag ctgcacaacc tctactacgc cccaattcag   9480
   caggaagcag ttagagcggt cgtcggccaa cctccccaac agcacttagg ttttcctgtt   9540
   gagatggggg actgagagac aggactagct ggatttccta ggctgactaa gaatccctaa   9600
   gcctagctgg gaaggtgacc acatccacct ttaaacacgg ggcttgcaac ttagctcaca   9660
   cctgaccaat cagagagctc actaaaatgc taattaggca aagacaggag gtaaagaaat   9720
   agccaatcat ctattgcctg agagcacagc aggagggaca atgatcggga tataaaccca   9780
   agtcttcgag ccggcaacgg caaccccctt tgggtcccct ccctttgtat gggagctctg   9840
   ttttcatgct atttcactct attaaatctt gcaactgcac tcttctggtc catgtttctt   9900
   acggcttgag ctgagctttc gctcgccatc caccactgct gtttgccgcc accgcagacc   9960
   cgccgctgac tcccatccct ctggatcatg cagggtgtcc gctgtgctcc tgatccagcg   10020
   aggcacccat tgccgctccc aatcgggcta aaggcttgcc attgttcctg catggctaag   10080
   tgcctgggtt catcctaatt gagctgaaca ctagtcactg ggttccatgg ttctcttctg   10140
   tgacccacag cttctaatag agctataaca ctcaccgcat ggcccaaggt tccattcctt   10200
   gaatccataa ggccaagaac cccaggtcag agaacacgag gcttgccacc atcttgggag   10260
   ctctgtgagc aaggaccccc aagtaaca                                      10288
<210> 14
   <211> 18
   <212> DNA
   <213> Artificial
<220>
   <223> amorce d'amplification
<400> 14
   tgcagatgct gtgtctgg 18
<210> 15
   <211> 17
   <212> DNA
   <213> Artificial
<220>
   <223> amorce d'amplification
<400> 15
   cgtactggcc caggacc 17
<210> 16
   <211> 20
   <212> DNA
   <213> Artificial
<220>
   <223> amorce d'amplification
<400> 16
   ggttcgtgct aattgagctg 20
<210> 17
   <211> 20
   <212> DNA
   <213> Artificial
<220>
   <223> amorce d'amplification
<400> 17
   atggtggcaa gcttcttgtt 20
<210> 18
   <211> 20
   <212> DNA
   <213> Artificial
<220>
   <223> amorce d'amplification
<400> 18
   tgagctttcc ctcactgtcc 20
<210> 19
   <211> 20
   <212> DNA
   <213> Artificial
<220>
   <223> amorce d'amplification
<400> 19
   tgttcggctt gattaggatg 20
<210> 20
   <211> 20
   <212> DNA
   <213> Artificial
<220>
   <223> amorce d'amplification
<400> 20
   catggcccaa tattccattc 20
<210> 21
   <211> 21
   <212> DNA
   <213> Artificial
<220>
   <223> amorce d'amplification
<400> 21
   ggtccttgtt cacagaactc c 21
<210> 22
   <211> 20
   <212> DNA
   <213> Artificial
<220>
   <223> amorce d'amplification
<400> 22
   ccgctcctga ttggactaaa 20
<210> 23
   <211> 20
   <212> DNA
   <213> Artificial
<220>
   <223> amorce d'amplificaton
<400> 23
   cgtgggtcaa ggaagagaac 20
<210> 24
   <211> 21
   <212> DNA
   <213> Artificial
<220>
   <223> amorce d'amplification
<400> 24
   atgacccgca gcttctaaca g 21
<210> 25
   <211> 20
   <212> DNA
   <213> Artificial
<220>
   <223> amorce d'amplification
<400> 25
   ctccgctcac agagctccta 20
<210> 26
   <211> 21
   <212> DNA
   <213> Artificial
<220>
   <223> amorce d'amplification
<400> 26
   ccaacatcac taacacaacc t 21
<210> 27
   <211> 20
   <212> DNA
   <213> Artificial
<220>
   <223> amorce d'amplification
<400> 27
   gggagttagt aaggggtttg 20
<210> 28
   <211> 24
   <212> DNA
   <213> Artificial
<220>
   <223> amorce d'amplification
<400> 28
   caacctatta aacaaaacta aatt 24
<210> 29
   <211> 27
   <212> DNA
   <213> Artificial
<220>
   <223> amorce d'amplification
<400> 29
   agatttaata gagtgaaaat agagttt 27
<210> 30
   <211> 22
   <212> DNA
   <213> Artificial
<220>
   <223> amorce d'amplification
<400> 30
   ttattagttt aggggatagt tg 22
<210> 31
   <211> 23
   <212> DNA
   <213> Artificial
<220>
   <223> amorce d'amplification
<400> 31
   acacaataaa caacctacta aat 23
<210> 32
   <211> 19
   <212> DNA
   <213> Artificial
<220>
   <223> amorce d'amplification
<400> 32
   gagggtaagt ggtgataaa 19
<210> 33
   <211> 21
   <212> DNA
   <213> Artificial
<220>
   <223> amorce d'amplification
<400> 33
   aacctactaa atccaaaaaa a 21
<210> 34
   <211> 22
   <212> DNA
   <213> Artificial
<220>
   <223> amorce d'amplication
<400> 34
   taggatttta ggtttattgt ta 22
<210> 35
   <211> 19
   <212> DNA
   <213> Artificial
<220>
   <223> amorce d'amplification
<400> 35
   aaaaataaaa tattaaacc 19
<210> 36
   <211> 20
   <212> DNA
   <213> Artificial
<220>
   <223> amorce d'amplification
<400> 36
   atatgtggga gtgagagata 20
<210> 37
   <211> 22
   <212> DNA
   <213> Artificial
<220>
   <223> amorce d'amplification
<400> 37
   caacaacaaa caataataat aa 22
<210> 38
   <211> 22
   <212> DNA
   <213> Artificial
<220>
   <223> amorce d'amplification
<400> 38
   ttgagttttt ttattgatag tg 22
<210> 39
   <211> 21
   <212> DNA
   <213> Artificial
<220>
   <223> amorce d'amplification
<400> 39
   tctaaatcct attttcctac t 21
<210> 40
   <211> 24
   <212> DNA
   <213> Artificial
<220>
   <223> amorce d'amplification
<400> 40
   gtttttttat tgatagtgag agat 24
<210> 41
   <211> 20
   <212> DNA
   <213> Artificial
<220>
   <223> amorce d'amplification
<400> 41
   taacaaacct ttaatccaat 20
<210> 42
   <211> 22
   <212> DNA
   <213> Artificial
<220>
   <223> amorce d'amplification
<400> 42
   tttagtgagg atgatgtaat at 22
<210> 43
   <211> 22
   <212> DNA
   <213> Artificial
<220>
   <223> amorce d'amplification
<400> 43
   caacttaata aaaataaacc ca 22
<210> 44
   <211> 24
   <212> DNA
   <213> Artificial
<220>
   <223> amorce d'amplification
<400> 44
   ataatgtttt agtaagtgtt ggat 24
<210> 45
   <211> 20
   <212> DNA
   <213> Artificial
<220>
   <223> amorce d'amplification
<400> 45
   acaattacaa acctttaacc 20
<210> 46
   <211> 20
   <212> DNA
   <213> Artificial
<220>
   <223> amorce d'amplification
<400> 46
   aattcattca acatccattc 20
<210> 47
   <211> 26
   <212> DNA
   <213> Artificial
<220>
   <223> amorce d'amplification
<400> 47
   ggtttaatat tatttattat tttgga 26
<210> 48
   <211> 25
   <212> DNA
   <213> Artificial
<220>
   <223> amorce d'amplification
<400> 48
   ctcttacctt cctatactct ctaaa 25
<210> 49
   <211> 28
   <212> DNA
   <213> Artificial
<220>
   <223> amorce d'amplification
<400> 49
   agagtgtagt tgtaagattt aatagagt 28